# EUROPEAN PATENT SPECIFICATION

(11) **EP 4 240 734 B1**
(45) Date of publication and mention of the grant of the patent: **18.02.2026**
(21) Application number: 21810704.3
(22) Date of filing: 03.11.2021
(51) Int. Cl.: C07D 401/14, C07D 403/14, C07D 413/14, C09K 11/06

(54) **ORGANIC COMPOUNDS FOR USE IN ELECTROLUMINESCENT DEVICES**
ORGANISCHE VERBINDUNGEN ZUR VERWENDUNG IN ELEKTROLUMINESZENTEN VORRICHTUNGEN
COMPOSÉS ORGANIQUES DESTINÉS À ÊTRE UTILISÉS DANS DES DISPOSITIFS ÉLECTROLUMINESCENTS

(30) Priority: 03.11.2020 GB 202017406
(43) Date of publication of application: 13.09.2023
(73) Proprietor: University Court of The University of St Andrews, St Andrews, Fife KY16 9AJ (GB)
(72) Inventor: ZYSMAN-COLMAN, Eli, St. Andrews Fife KY16 9ST (GB); SUN, Dianming, St. Andrews Fife KY16 9ST (GB)
(74) Representative: Marks & Clerk LLP
(86) International application number: PCT/GB2021/052844
(87) International publication number: WO 2022/096869

(56) References cited:
- EP-A1- 3 670 507
- WO-A1-2015/137472
- CN-A- 109 111 433

## Description

### FIELD OF THE INVENTION

The invention relates to thermally activated delayed fluorescence (TADF) dendrimers that are useful in electroluminescent devices such as organic light emitting diode (OLED)s. The invention extends to OLEDs comprising one or more of these compounds as an emitter material.

### BACKGROUND OF THE INVENTION

The broadcasting of information in modern society is heavily dependent on displays and OLEDs are becoming increasingly desirable for curved TVs, high-contrast-ratio smart phones and virtual reality (VR) technologies. In comparison with traditional liquid crystal displays (LCDs), OLEDs have been found to provide improved image quality (better contrast, higher brightness, fuller viewing angle, a wider color range and much faster refresh rates), have lower power consumption, and can be of simpler design (and thus more suited to enabling ultra-thin, flexible, foldable and transparent displays).

OLEDs are light emitting diodes in which the emissive electroluminescent layer is a film of an organic compound or of organic compounds that emits light in response to an electric current. In this context, an organic compound is a compound that contains carbon covalently bond to other atoms, especially carbon bonded to carbon (C-C) and carbon bonded to hydrogen (C-H). Such organic compounds include small molecules and dendrimers. Unlike small molecule emitters, which are apt to crystalize during operation, dendrimers are branched macromolecules in which branched dendrons are attached to a core structure. At present vacuum deposition technology is widely adopted for the fabrication of organic small molecule and some dendrimer-based OLEDs. For example, CN 109111433 A discloses organic electroluminesent compounds which, in combination with a host material, are deposited on a hole transport layer using vacuumdeposition, to form the light-emitting layer of an OLED. This technology is problematic as a significant amount of material is wasted because it is dispersed all over the mask, in addition to inherent mask changes that can compromise yields. The intrinsic properties of dendrimers can make them suitable candidates for solution-processing methods such as ink-jet printing and roll-to-roll processing, and thus they can realize special application on flexible, foldable displays. Further, solution processing can result in the production of OLEDs at lower cost, higher speed and in higher volumes. There is a need for new solution-processable dendrimers suitable for use as emitting material in OLEDs.

A parameter used to characterise an OLED is its external quantum efficiency (EQE), the ratio of the number of photons emitted from the OLED to the number of electrons passing through the device. The EQE is a measure of how efficiently the device converts electrons to photons and allows them to escape. It is desirable for an OLED to have a high EQE. There is a need for new emitting materials that can enable OLEDs achieve a high EQE.

TADF materials have performance advantages over, e.g. conventional fluorescent materials since they can harvest both singlet and triplet excitons for light emission and thus can have a high internal quantum efficiency (up to 100 %). TADF dendrimers for solution-processed OLEDs are reported in Albrecht, K. et al. "Dendrimers as Solution-Processable Thermally Activated Delayed-Fluorescence Materials", Angew. Chem. Int. Ed. 2015, 54 (19), 5677-5682. However, the most efficient device based on a green TADF dendrimer achieved a maximum external efficiency (EQEₘₐₓ) of 16.1%. This EQEₘₐₓ performance lags behind that of OLEDs based on small molecules, which have already reached more than 30% for EQEₘₐₓ. Further, this device requires a host-guest configuration, i.e. the dendrimer (guest) is doped into a suitable host. The host material dilutes the concentration of the guest material and acts to suppress concentration quenching and triplet-triplet annihilation. Host-guest configurations attract the following problems: i) batch-to-batch processing issues relevant to the reproducibility of performance of the devices that is necessary for commercialization; ii) an increase in the fabrication cost of the device, especially as there is a need to finely control the doping ratio by co-evaporation; iii) phase separation during operation of devices, which is detrimental for long term stability of the device; iv) high cost and poor sustainability associated with known phosphorescent dendrimers based on noble metal complex core structures; and v) low efficiency that has been found for devices using conventional organic fluorescent dendrimers. There is a need for new TADF dendrimers that can act as emitting material in an OLED. In particular, there is a need for new TADF dendrimers that can act as an emitting material without being doped in a host material, i.e. that can suppress concentration and triplet-triplet annihilation in the absence of a hot material.

It is an objective of the present invention to meet one or more of the needs or solve one or more of the problems described above.

### SUMMARY OF THE INVENTION

In a first aspect, the invention provides a compound of formula (I),

(Ar₁)ₘ-(Ar₂)-(X)ₙ (I)

wherein:
Ar₂ is a pyridine, pyrimidine, pyrazine, pyridazine, triazine or tetrazine ring group;
m is from 1 to 5;
n is 0, 1 or 2;
each Ar₁ is independently selected from the following: 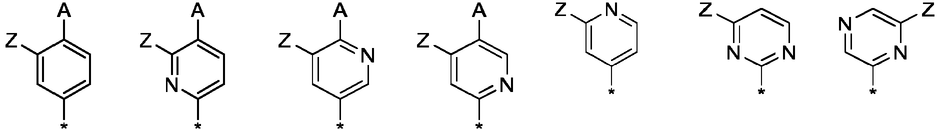 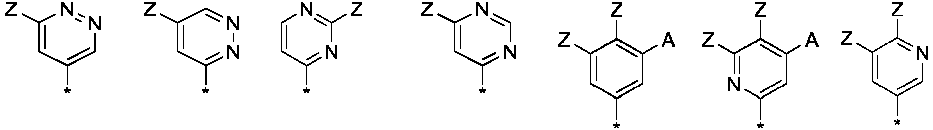 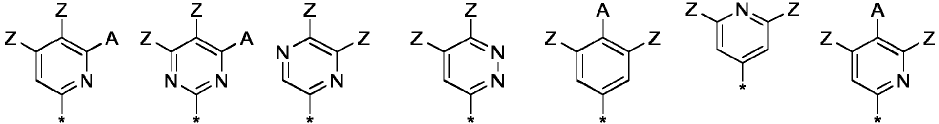 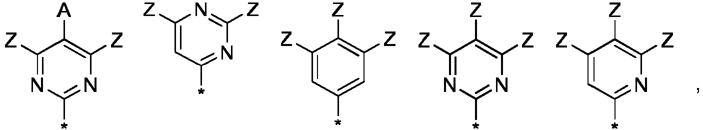
wherein each Z is independently selected from Z2 to Z4: 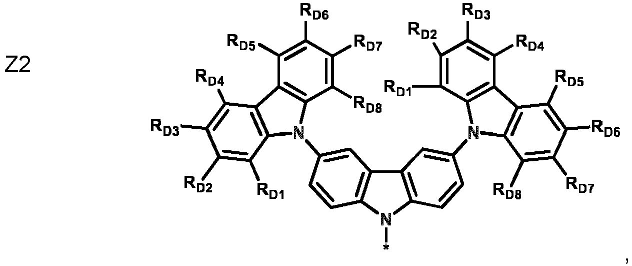 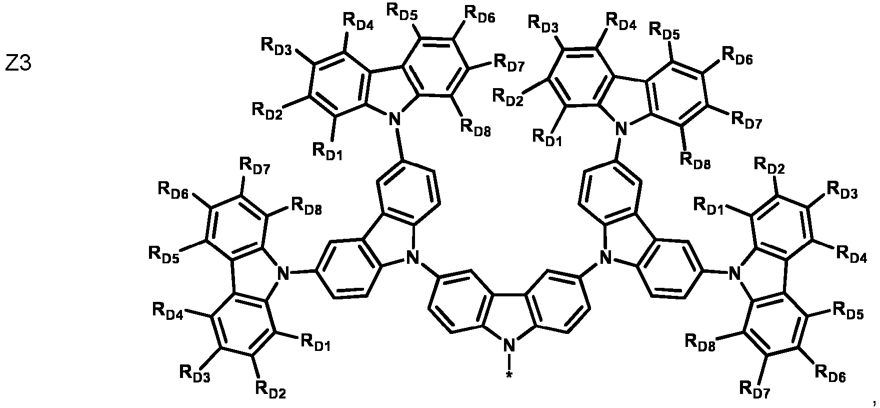 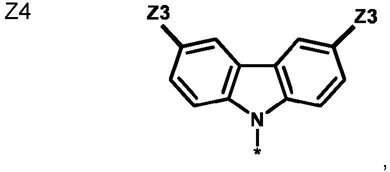
wherein R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl,
wherein R_{D2}, R_{D3}, R_{D6} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q,
wherein Q is selected from the following: 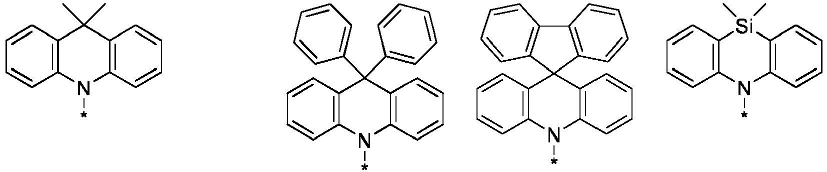 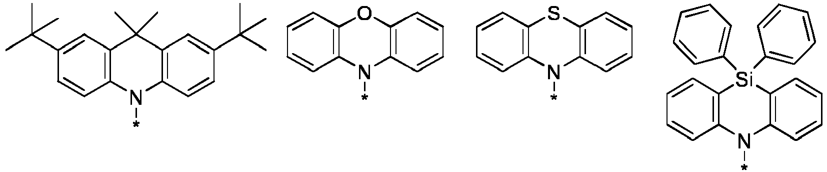 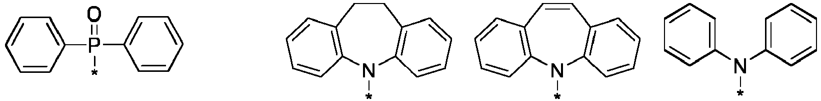 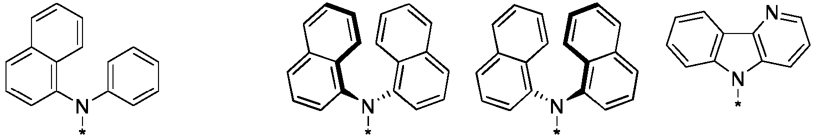 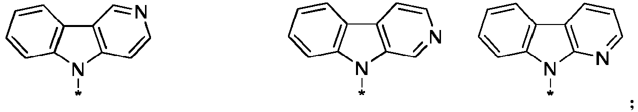
wherein A is selected from hydrogen, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy and silyl;
wherein each X is independently selected from substituted or unsubstituted alkyl, substituted or unsubstituted cycloalkyl, and the following: 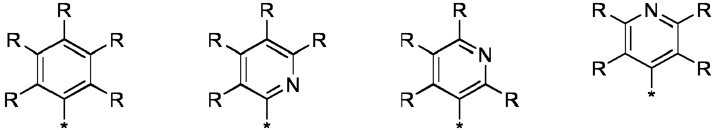 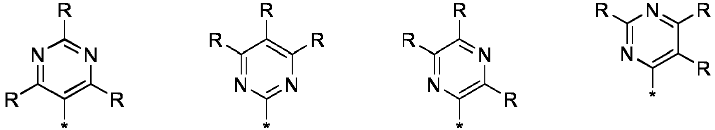 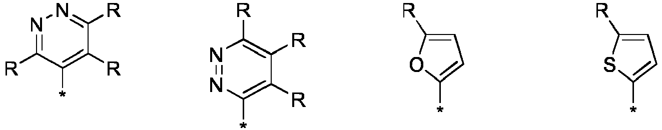 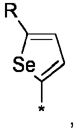
wherein each R is independently selected from the group consisting of hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and diphenylphosphine oxide; and
wherein * represents a point of attachment; and
wherein for Z2, each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q.

In another aspect, the invention provides an OLED comprising an emissive layer, wherein the emissive layer comprises one or more compounds according to the first aspect of the invention. The one or more compounds act as an emitter material in the emissive layer.

In a further aspect, the invention provides a method of preparing an OLED, comprising depositing an emissive layer on a substrate using a solution processing technique, wherein said emissive layer comprises one or more compounds according to the first aspect of the invention.

### DETAILED DESCRIPTION OF THE INVENTION

The present invention relates to TADF dendrimers that are useful as emitter material for OLEDs. The invention is based, in part, on the recognition that the compounds (dendrimers) of formula (I) described above are solution-processable, e.g. they exhibit excellent solubility in commonly used solvents, and are suitable for use as emitting materials in OLEDs. Advantageously, compounds of the invention can be used non-doped in OLEDs, i.e. where they do not require a host material to suppress concentration quenching and triplet-triplet annihilation and are present in the emissive layer in the absence of any host materials. It has been found that compounds of the invention can provide OLEDs with high external quantum efficiency (EQE) values, even when used in non-doped form.

In a first aspect, the invention relates to compounds of formula (I), (Ar₁)ₘ-(Ar₂)-(X)ₙ, as described above. The compounds of the invention are also referred herein as dendrimers. The compounds of the invention are metal-free, i.e. they do not contain any metal atoms.

The compound (dendrimer) of formula (I) comprises an acceptor core Ar₂ linked to one or more donor Z groups, wherein each Z group is linked to the acceptor core Ar₂ via a six-membered aromatic or heteroaromatic ring. Z is referred to herein as a dendron, a donor dendron, a Z group, a dendron Z, or a Z dendron. The six-membered aromatic or heteroaromatic ring is also referred herein as a linking six-membered aromatic or heteroaromatic ring or, simply, as a linking ring. The linking ring provides a conjugated or π bridge between the acceptor core Ar₂ and the donor dendron(s). Ar₁ represents each linking ring and its associated Z dendrons. An important feature of the invention is that the compound of formula (I) comprises at least one dendron Z that is meta-connected to the acceptor core Ar₂, i.e. it comprises at least one Ar₁ group whereby the dendron Z is attached to a position on the linking ring that is meta to the position of attachment to Ar₂ on said ring. In other words, a meta-connected dendron Z is a dendron Z that occupies a ring position on Ar₁ that is meta to the ring position of attachment of Ar₁ to Ar₂. By position on the linking ring of Ar₁ or ring position on Ar₁ is meant a carbon atom that is one of the carbon atoms that forms the linking ring of Ar₁.

In particular, it has been found that dendrimers of the invention can have performance advantages over TADF dendrimers that are based solely on para-connected conjugated bridges. The incorporation of meta-connected conjugated bridges is believed to provide an avenue for a TADF dendrimer possessing a small ΔE_{ST} as the fragment donor and acceptor groups are electronically decoupled. Further, increasing the number of dendrons and the density with which they are packed together is believed to suppress quenching on the core units caused by intramolecular interactions. Also, the presence of a large number of donor dendrons surrounding the central acceptor is believed to lead to an increase in the density of triplet states that will enhance the nonadiabatic effect leading to more efficient Reverse Inter-System Crossing (RISC) between T₁ and S₁. Advantageously, the compounds of the invention can contain from 2 to 6, or 4 to 6 Z dendrons.

### Ar₂

The acceptor core Ar₂ is a pyridine, pyrimidine, pyrazine, pyridazine, triazine or tetrazine ring. Ar₂ can be a pyridine, pyrimidine, pyrazine, triazine or tetrazine ring. Ar₂ can be a pyridine, pyrimidine, pyrazine or pyridazine ring. Ar₂ can be a triazine or tetrazine ring. By triazine is meant 1,3,5-triazine and by tetrazine is meant 1,2,3,4-tetrazine ring. In an exemplary embodiment, the acceptor core Ar₂ is a 1,3,5-triazine ring. In an exemplary embodiment, the acceptor core Ar₂ is a 1,3,5-triazine ring. The acceptor core Ar₂ is substituted by at least one Ar₁ group and, optionally, one or two X groups. Each of Ar₁ and X is a substituent on a ring carbon atom of Ar₂. A ring carbon is one of the carbon atoms that forms the ring of Ar₂.

### m

The number of Ar₁ groups attached to the acceptor core Ar₂ is m, where m is from 1 to 5. Thus, the acceptor core Ar₂ has at least one Ar₁ group attached. Preferably, the acceptor core has two or more Ar₁ groups attached; for example, m can be 2 or 3. When the acceptor core has two or more Ar₁ groups attached, for example, when m is 2 or 3, at least one of the Ar₁ groups can be different from the other Ar₁ groups(s). Alternatively, when the acceptor core has two or more Ar₁ groups attached, for example, when m is 2 or 3, the Ar₁ groups can be the same. The maximum number of Ar₁ groups that can be attached to the acceptor core Ar₂ is the total number of ring carbon atoms in Ar₂. Thus when Ar₂ is pyridine, m is from 1 to 5; when Ar₂ is pyrimidine, m is from 1 to 4; when Ar₂ is pyrazine, m is from 1 to 4; when Ar₂ is pyridazine, m is from 1 to 4; when Ar₂ is triazine, m is from 1 to 3; and when Ar₂ is tetrazine, m is 1 or 2.

### n

The number of X groups attached to the acceptor core Ar₂ is n, where n is 0, 1, or 2. The sum of m and n can be the total number of ring carbon atoms of Ar₂, and when this is the case, each carbon atom of Ar₂ is substituted with either Ar₁ or X. The sum of m and n can be less than the total number of ring carbon atoms of Ar₂, and when this is the case, at least one of the ring carbon atoms of Ar₂ is unsubstituted by Ar₁ or X, i.e. it is attached to hydrogen. When Ar₂ is pyridine, n can be 0, 1 or 2 ; when Ar₂ is pyrimidine, n can be 0, 1 or 2; when Ar₂ is pyrazine, n can be 0, 1 or 2; when Ar₂ is pyridazine, n can be 0, 1 or 2; when Ar₂ is triazine, n can be 0, 1 or 2; and when Ar₂ is tetrazine, n can be 0 or 1.

### Ar₁

Ar₁ is a six-membered aromatic or heteroaromatic ring comprising at least one dendron Z positioned meta to the ring position on Ar₁ where Ar₁ is attached to acceptor core Ar₂. Specifically, each Ar₁ is independently selected from the following:

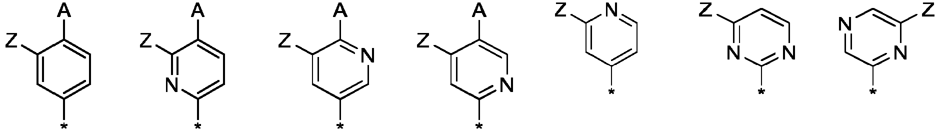

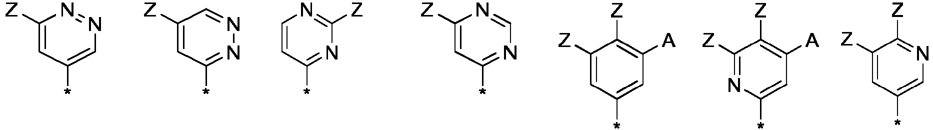

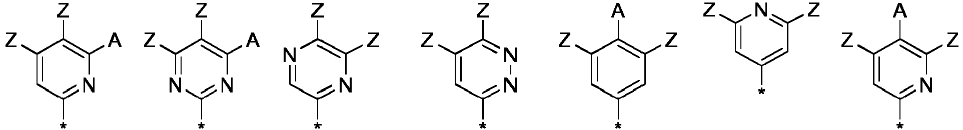

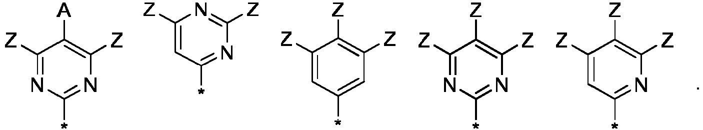

In the structures given above for Ar₁, * represents a point of attachment to Ar₂. Each Ar₁ has one, two or three Z dendrons attached. Each Ar₁ can have one or two Z dendrons attached and can be independently selected from the following:

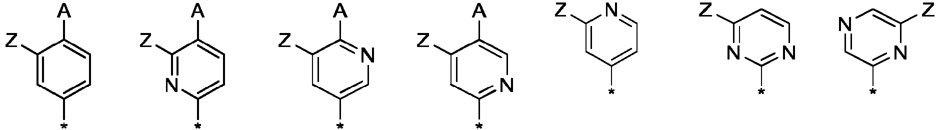

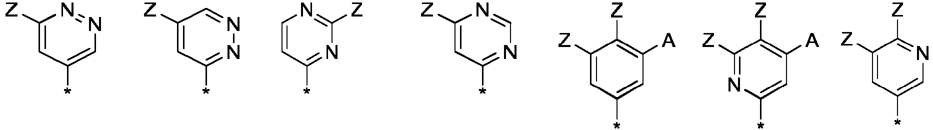

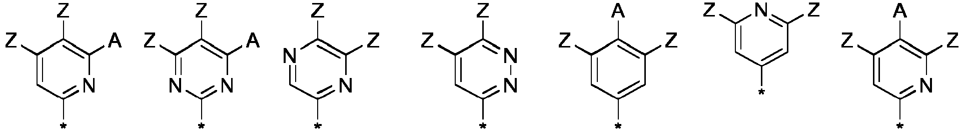

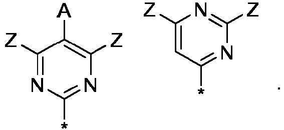

Each Ar₁ can have two or three Z dendrons attached and can be independently selected from the following:

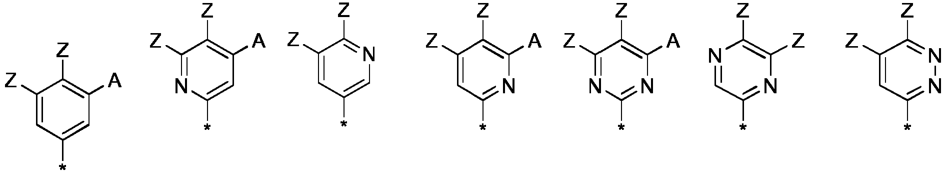

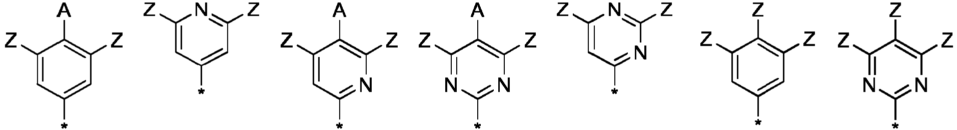

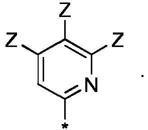

Each Ar₁ can have two Z dendrons attached and can be independently selected from the following:

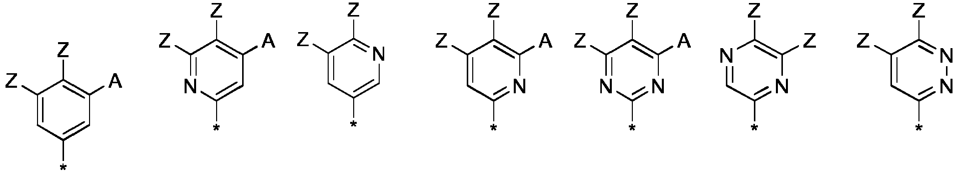

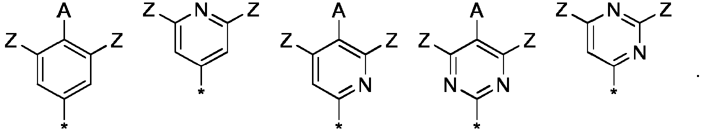

The Z dendrons occupy ring positions on Ar₁ that are either meta or para to the position of attachment to Ar₁ to acceptor core Ar₂ (with the proviso that at least one Z dendron occupies a ring position that is meta to the point of attachment of Ar₁ to acceptor core Ar₂). The importance of the presence of a meta-connected dendron Z is discussed above.

### Z

Each Z dendron is independently chosen from Z2, Z3 and Z4 as described above and discussed further below. Thus, for an Ar₁ that has one dendron Z attached, Z is selected from Z2, Z3 and Z4. Thus, for an Ar₁ that has two or three dendrons Z attached, each Z is independently selected from Z2, Z3 and Z4. Each Z dendron can be Z2. For an Ar₁ that has two or three Z dendrons attached to it, at least one dendron Z can be different from the other dendron(s) Z. Thus, for an Ar₁ that has two Z dendrons attached to it, each Z dendron is different, and for an Ar₁ that has three Z dendrons attached to it, either (i) two of the Z dendrons are the same and one of the Z dendrons is different from the other two Z or (ii) each of the Z dendrons is different. Alternatively, for an Ar₁ that has two or three Z dendrons attached to it, each of the dendrons Z can be the same.

### Z2

Z2 is 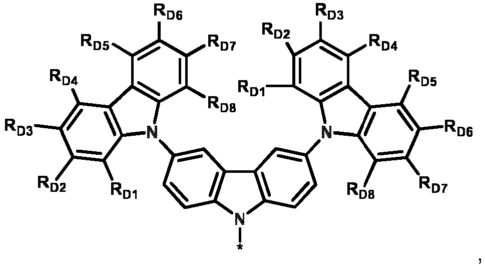 wherein * represents a point of attachment to Ar₁. Z2 is a dendron comprising two tiers of carbazole groups. R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl. R_{D2} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. Each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. At least one of R_{D2}, R_{D3}, R_{D6} and R_{D7} may be Q.

The second tier carbazole groups are substituted at the 3 and 6 positions (R_{D3} and R_{D6}). Thus: R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl; R_{D2} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q; and R_{D3} and R_{D6} are each independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, R_{D3} and R_{D6} are each independently selected from substituted or unsubstituted alkyl, for example a C1 to C6 alkyl, such as tertiary butyl. R_{D3} and R_{D6} can be different. Alternatively, R_{D3} and R_{D6} can be the same. In one embodiment, R_{D3} and R_{D6} are each tertiary butyl. In one embodiment, each of R_{D1} and R_{D8} is hydrogen and, preferably, each of R_{D1}, R_{D2}, R_{D4}, R_{D5}, R_{D7} and R_{D8} is hydrogen. In an exemplary embodiment, each of R_{D3} and R_{D6} is tertiary butyl and each of R_{D1}, R_{D2}, R_{D4}, R_{D5}, R_{D7} and R_{D8} is hydrogen.

### Z3

Z3 is 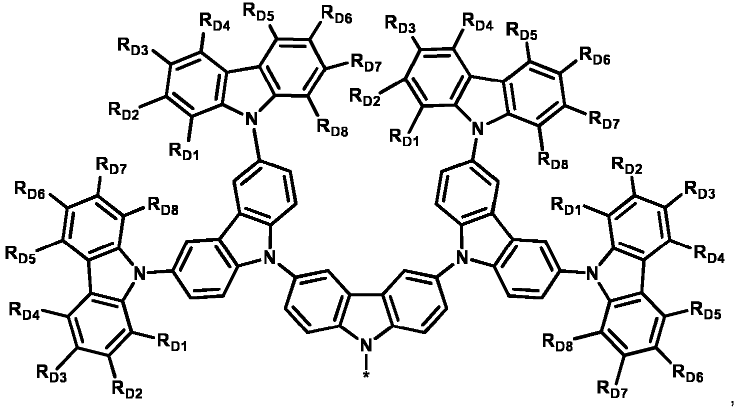 where * represents either a point of attachment to Ar₁ or a point of attachment within Z4. Z3 is a dendron comprising three tiers of carbazole groups. R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl. R_{D2}, R_{D3}, R_{D6} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, R_{D3} and R_{D6} are each independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. Each of R_{D3} and R_{D6} can be selected from substituted or unsubstituted alkyl, for example a C1 to C6 alkyl such as tertiary butyl. R_{D3} and R_{D6} can be different. Alternatively, R_{D3} and R_{D6} can be the same. In one embodiment, R_{D3} and R_{D6} are each tertiary butyl. Each of R_{D1} and R_{D8} can be hydrogen and, preferably, each of R_{D1}, R_{D2}, R_{D4}, R_{D5}, R_{D7} and R_{D8} is hydrogen. In an exemplary embodiment, each of R_{D3} and R_{D6} is tertiary butyl and each of R_{D1}, R_{D2}, R_{D4}, R_{D5}, R_{D7} and R_{D8} is hydrogen.

### Z4

Z4 is 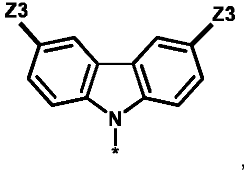 where * represents either a point of attachment to Ar₁. Z4 is a dendron comprising four tiers of carbazole groups and its full structure is shown in Figure 1 R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl. R_{D2}, R_{D3}, R_{D6} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, R_{D3} and R_{D6} are each independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. Each of R_{D3} and R_{D6} can be selected from substituted or unsubstituted alkyl, for example a C1 to C6 alkyl such as tertiary butyl. R_{D3} and R_{D6} can be different. Alternatively, R_{D3} and R_{D6} can be the same. In one embodiment, R_{D3} and R_{D6} are each tertiary butyl. Each of R_{D1} and R_{D8} can be hydrogen and, preferably, each of R_{D1}, R_{D2}, R_{D4}, R_{D5}, R_{D7} and R_{D8} is hydrogen. In an exemplary embodiment, each of R_{D3} and R_{D6} is tertiary butyl and each of R_{D1}, R_{D2}, R_{D4}, R_{D5}, R_{D7} and R_{D8} is hydrogen.

### A

Certain Ar₁ groups comprise an A substituent, as indicated in the structures for Ar₁ described above. A is selected from hydrogen, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy and silyl. For example, A can be selected from hydrogen, CN, tetrazole, or a substituted diazole.

In some embodiments, when Ar₁ is any of the following structures:

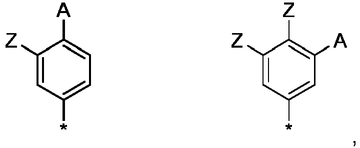

A is selected from hydrogen, halo, ester, nitro, cyano, haloalkyl, sulfonyl, carbonyl, substituted or unsubstituted alkyl, substituted or unsubstituted alkoxy, substituted or unsubstituted aryloxy, substituted or unsubstituted heteroaryl, substituted or unsubstituted heteroaryloxy and silyl.

In some embodiments, when Ar₁ is any of the following structures:

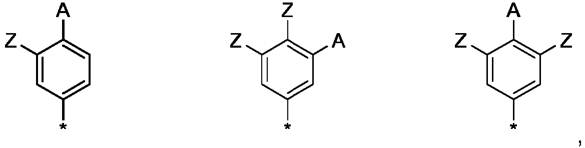

A is selected from hydrogen, halo, ester, nitro, cyano, haloalkyl, sulfonyl, carbonyl, substituted or unsubstituted alkyl, substituted or unsubstituted alkoxy, substituted or unsubstituted aryloxy, substituted or unsubstituted heteroaryl, substituted or unsubstituted heteroaryloxy and silyl.

Ar₁ can be selected from: 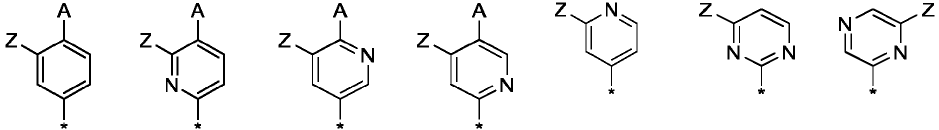 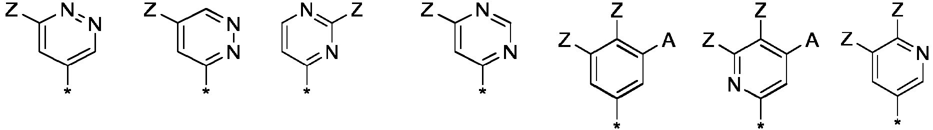 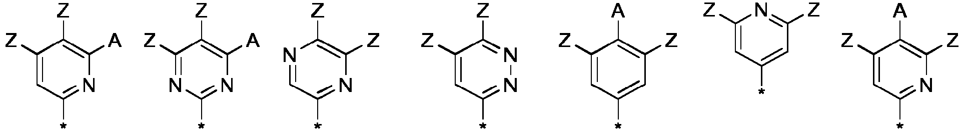 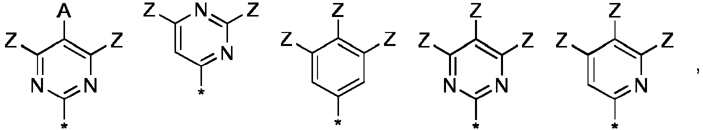
wherein A is selected from hydrogen, CN, tetrazole, or a substituted diazole. In one embodiment A is hydrogen. Ar₁ can be selected from: 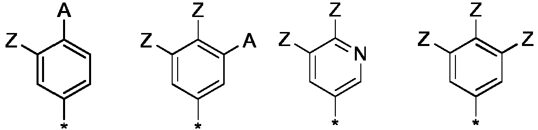
wherein A is hydrogen and 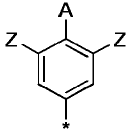
wherein A is hydrogen, CN, tetrazole, or a substituted diazole.

### X

Each X group is independently chosen from substituted or unsubstituted alkyl, substituted or unsubstituted cycloalkyl, and the following groups: 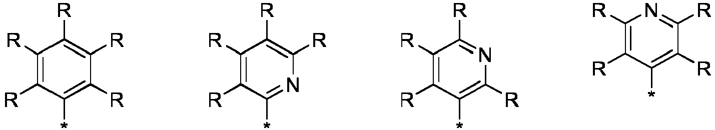 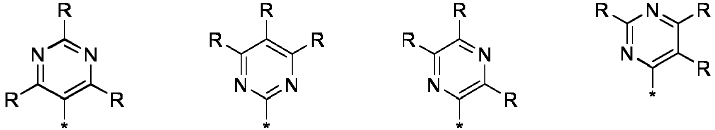 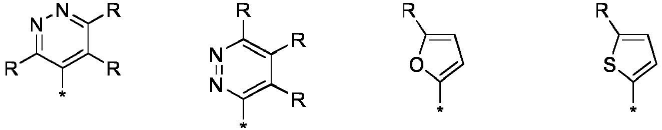 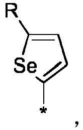 wherein each R is independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and diphenylphosphine oxide. X can be a substituted or unsubstituted polycycloalkyl, such as the radical of bicyclobutane, bicyclohexane, bicycloheptane, bicyclooctane, bicyclononane, tricyclobutane, tetracyclopentane, pentacyclopentane, heptocyclohexane, prismane and adamantane (shown below).

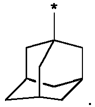

X can be phenyl, i.e. have the structure. 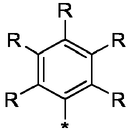 where each R is hydrogen.

### Number of Z

The number of Z dendrons in formula (I) depends on the Ar₁ groups (which can have one, two or three Z dendrons each) and the value of m (which is the number of Ar₁ groups). Preferably, the number of Z dendrons is greater than 2. The number of dendrons can be from 1 to 6 and is preferably from 2 to 6. The number of dendrons can be from 2 to 4 when Ar₂ is triazine or tetrazine, and the number of dendrons can be 5 or 6 when Ar₂ is pyridine, pyrimidine, pyrazine or pyridazine.

The compound of the invention can be described by a compound of formula (I) in which number of dendrons is from 2 to 6, m is 2 or 3 and, preferably, each of the dendrons Z is the same and/or each of the Ar₁ groups is the same. Preferably each of the dendrons is chosen from Z2. Each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, each of R_{D3} and R_{D6} is independently a substituted or unsubstituted alkyl (more preferably C1-C6 alkyl, even more preferably tertiary butyl) and each of R_{D1}, R_{D2}, R_{D4}, R_{D5}, R_{D7} and R_{D8} is hydrogen.

The number of Z dendrons in the compound of the invention can be from 2 to 4. In this embodiment, Ar₂ can be triazine or tetrazine.

When Ar₂ is a 1,3,5-triazine ring, the compound of the invention can be described by formula (la): 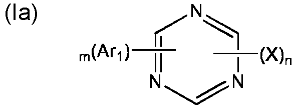 wherein m is 1 to 3, and n is 0, 1 or 2. Ar₁, m, X and n are as described above as for formula (I). When Ar₂ is a 1,2,3,4-tetrazine ring, the compound of the invention can be described by formula (Ib): 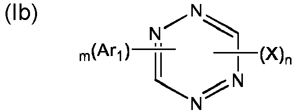 wherein m is 1 or 2, and n is 0 or 1. Ar₁, m, X and n are as described above as for formula (I).

The compound of the invention can be described by formula (la) or (Ib), where the number of Z dendrons is from 2 to 4 and m is 2 or 3. Preferably, each of the dendrons Z is the same. Each of the dendrons can be chosen from Z2. Each of R_{D3} and R_{D6} of Z2 is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, each of R_{D3} and R_{D6} is independently a substituted or unsubstituted alkyl (more preferably C1-C6 alkyl, even more preferably tertiary butyl) and each of R_{D1}, R_{D2}, R_{D4}, R_{D5}, R_{D7} and R_{D8} is hydrogen. In addition or alternatively (to each of the Z dendrons being the same), each of the Ar₁ groups can be the same. Preferably, each Ar₁ group is independently chosen from Ar₁ groups having one or two Z dendrons.

The compound of the invention can be described by formula (la) or (Ib), where the number of Z dendrons is 4. In particular, it is believed that the structure of 4 dendrimers connected to a triazine or tetrazine acceptor core is very effective in suppressing concentration quenching due to efficient encapsulation of the core structure by the dendrons. In one embodiment, the number of Z dendrons is 4, m is 2 and each Ar₁ is chosen from Ar₁ groups having two Z dendrons. In particular, the Z dendrons can be independently chosen from Z2. Preferably, each of the dendrons is the same and preferably each of the dendrons is chosen from Z2. Each of R_{D3} and R_{D6} of Z2 is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, each of R_{D3} and R_{D6} is independently a substituted or unsubstituted alkyl (more preferably C1-C6 alkyl, even more preferably tertiary butyl) and each of R_{D1}, R_{D2}, R_{D4}, R_{D5}, R_{D7} and R_{D8} is hydrogen. In addition or alternatively (to each of the Z dendrons being the same), each of the Ar₁ groups can be the same.

In one embodiment, the compound of the invention is described by formula (Ia) or (Ib), the number of Z dendrons is 4, m is 2, the Ar₁ groups are the same and are chosen from Ar₁ groups having two Z dendrons, the Z dendrons are the same and chosen from Z2. In particular in this embodiment, the compound of the invention can be described by formula (la). n can be 1 and, when n is 1, X can be a phenyl group. In particular, the Z dendrons are chosen from Z2.

In one embodiment, the number of Z groups is 5 or 6. In this embodiment, preferably Ar₂ is pyridine, pyrimidine, pyrazine or pyridazine. In particular, it is believed that the structure of 5 or 6 dendrimers connected to a pyridine, pyrimidine, pyrazine or pyridazine acceptor core is very effective in suppressing concentration quenching due to efficient encapsulation of core structure by dendrons.

When the acceptor core Ar₂ is a pyridine ring, the compound of the invention can be described by formula (Ic): 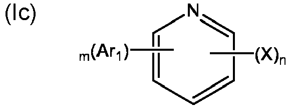 wherein m is 1 to 5 and n is 0, 1 or 2. Ar₁, m, X and n are as described above as for formula (I). When the acceptor core Ar₂ is a pyrimidine (1,3-diazine) ring, the compound of the invention can be described by formula (Id): 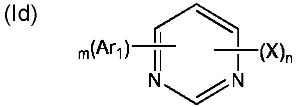 wherein m is 1 to 4 and n is 0, 1 or 2. Ar₁, m, X and n are as described above as for formula (I). When Ar₂ is a pyrazine (1,4-diazine) ring, the compound of the invention can be described by formula (le): 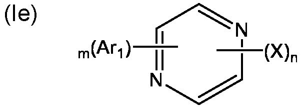 wherein m is 1 to 4 and n is 0, 1 or 2. Ar₁, m, X and n are as described above as for formula (I). When Ar₂ is a pyridazine (1,2-diazine) ring, the compound of the invention can be described by formula (If): 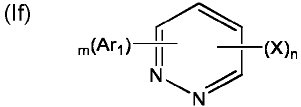 wherein m is 1 to 4 and n is 0, 1 or 2. Ar₁, m, X and n are as described above as for formula (I).

The compound of the invention can be described by formula (Ic), (Id) (le), or (If) where the number of Z dendrons is 5 or 6 and m is 2 or 3. Preferably each of the dendrons Z is the same. Each of the dendrons can be chosen from Z2. In particular, each of the dendrons can be chosen from Z2. Each of R_{D3} and R_{D6} of Z2 is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, each of R_{D3} and R_{D6} is independently a substituted or unsubstituted alkyl (more preferably C1-C6 alkyl, even more preferably tertiary butyl) and each of R_{D1}, R_{D2}, R_{D4}, R_{D5}, R_{D7} and R_{D8} is hydrogen. In addition or alternatively (to each of the Z dendrons being the same), each of the Ar₁ groups can be the same. Preferably, each Ar₁ group is independently chosen from Ar₁ groups having two or three Z dendrons.

The compound of the invention can be described by formula (Ic), (Id) (le), or (If) where the number of Z dendrons is 6, m is 2 and each Ar₁ is chosen from Ar₁ groups having three Z dendrons. Each of the dendrons Z can be the same and, preferably, each of the dendrons can be chosen from Z2. Each of R_{D3} and R_{D6} of Z2 is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In one embodiment, each of R_{D3} and R_{D6} is independently a substituted or unsubstituted alkyl (more preferably C1-C6 alkyl, even more preferably tertiary butyl) and each of R_{D1}, R_{D2}, R_{D4}, R_{D5}, R_{D7} and R_{D8} is hydrogen. In addition or alternatively (to each of the Z dendrons being the same), each of the Ar₁ groups can be the same.

In one embodiment, the compound of the invention can be described by formula (Ic), (Id) (le), or (If) the number of Z dendrons is 6, m is 2, the Ar₁ groups are the same and are chosen from Ar₁ groups having three Z dendrons, the Z dendrons are the same and chosen from Z2. In particular in this embodiment, the compound of the invention can be described by formula (Id). n can be 1 and, when n is 1, preferably X is a substituted or unsubstituted alkyl substituent, for example methyl.

The compound of formula (I) can be selected from the compounds shown in Table 1 below. In particular, the compound of formula (I) can be selected from the compounds I to XI shown in Table 1 below. The compound of formula (I) can be selected from the compounds I, II, III, IV and XI shown in Table 1 below. The compound of formula (I) can be selected from the compounds III and IV shown in Table 1 below.

**Table 1**

| | |
|---|---|
| I | 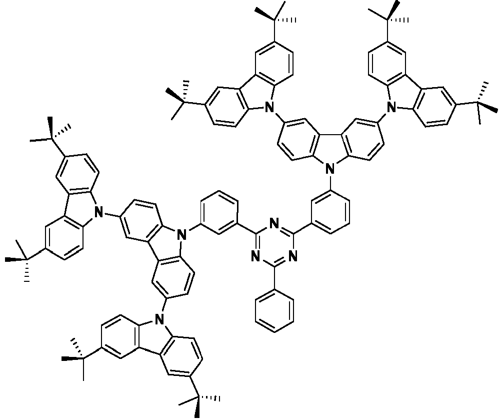 9',9ʺʺ-((6-phenyl-1,3,5-triazine-2,4-diyl)bis(3,1-phenylene))bis(3,3",6,6"-tetra-tert-butyl-9'H-9,3':6',9"-tercarbazole) (tBuCz2mTRZ) |
| II | 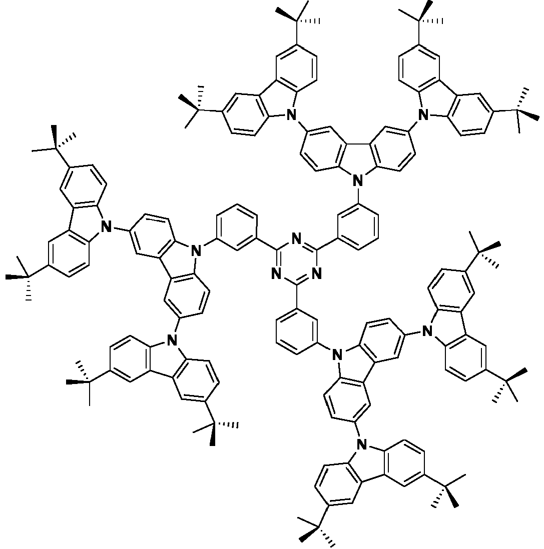 2,4,6-tris(3-(3,3",6,6"-tetra-tert-butyl-9'H-[9,3':6',9"-tercarbazol]-9'-yl)phenyl)-1,3,5-triazine (tBuCz3mTRZ) |
| III | 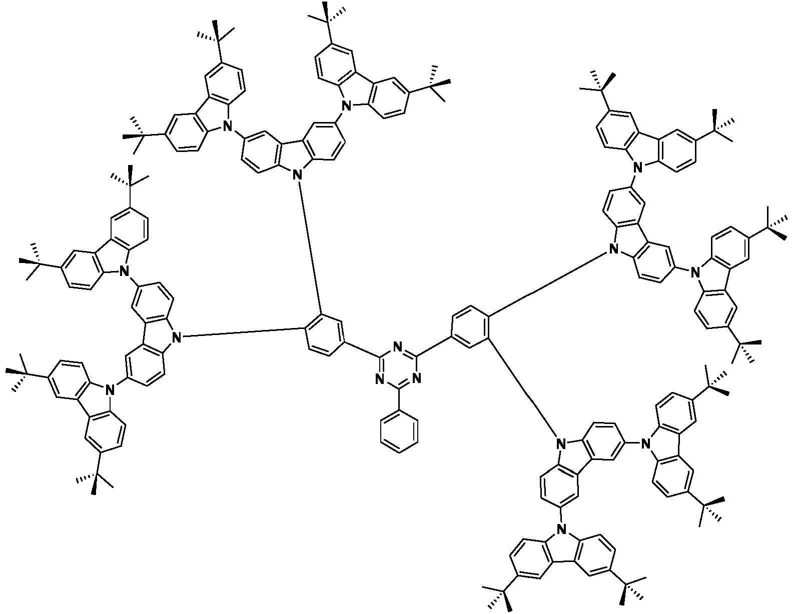 9',9ʺʺ,9‴ʺʺ,9‴‴ʺʺ-((6-phenyl-1,3,5-triazine-2,4-diyl)bis(benzene-4,1,2-triyl))tetrakis(3,3",6,6"-tetra-tert-butyl-9'H-9,3':6',9"-tercarbazole) (tBuCz2m2pTRZ) |
| IV | 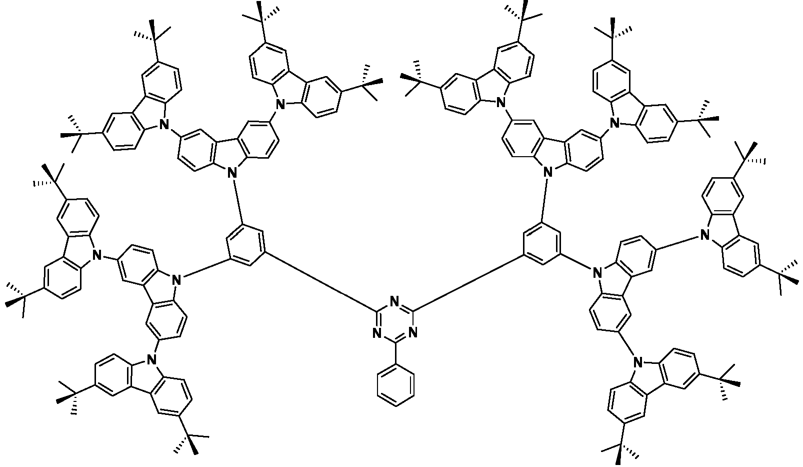 9',9ʺʺ,9‴ʺʺ,9‴‴ʺʺ-((6-phenyl-1,3,5-triazine-2,4-diyl)bis(benzene-5,1,3-triyl))tetrakis(3,3",6,6"-tetra-tert-butyl-9'H-9,3':6',9"-tercarbazole) (tBuCz4mTRZ) |
| V | 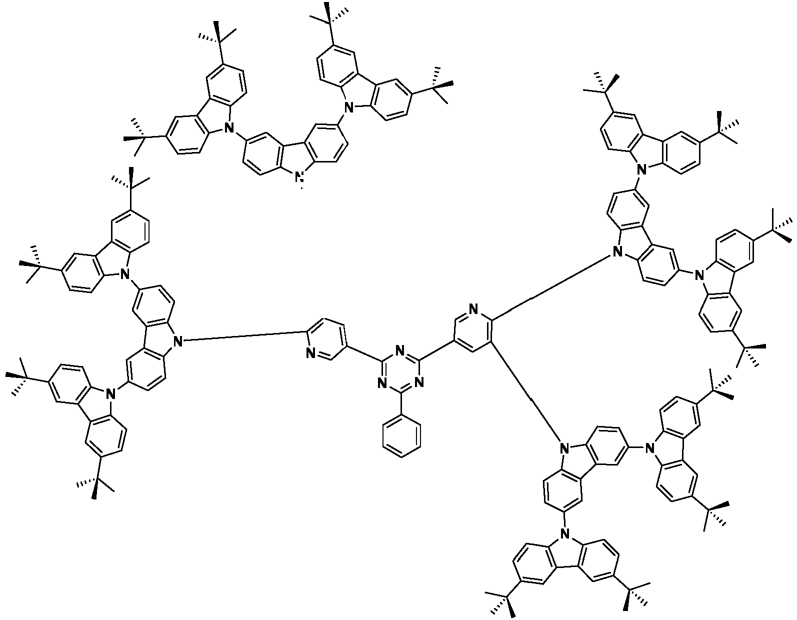 9',9ʺʺ,9‴ʺʺ,9‴‴ʺʺ-((6-phenyl-1,3,5-triazine-2,4-diyl)bis(pyridine-5,2,3-triyl))tetrakis(3,3",6,6"-tetra-tert-butyl-9'H-9,3':6',9"-tercarbazole) (tBuCz2m2pPyTRZ) |
| VI | 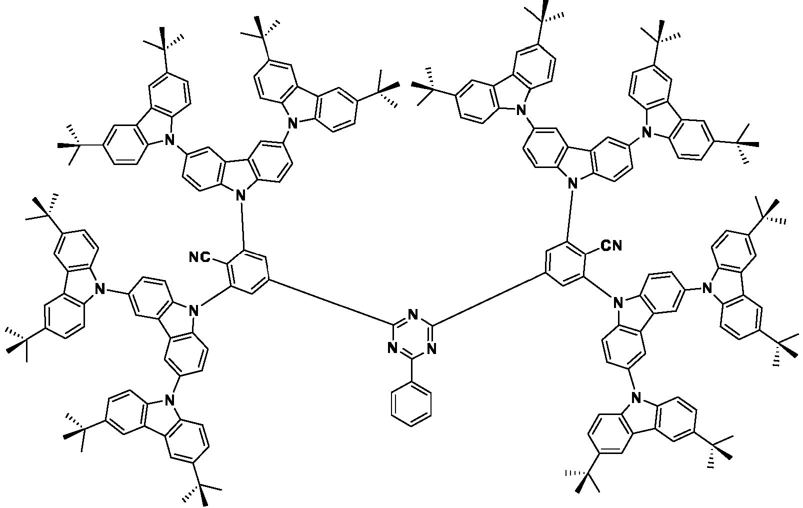 4,4'-(6-phenyl-1,3,5-triazine-2,4-diyl)bis(2,6-bis(3,3",6,6"-tetra-tert-butyl-9'H-[9,3':6',9"-tercarbazol]-9'-yl)benzonitrile) (tBuCz4m2CNTRZ) |
| VII | 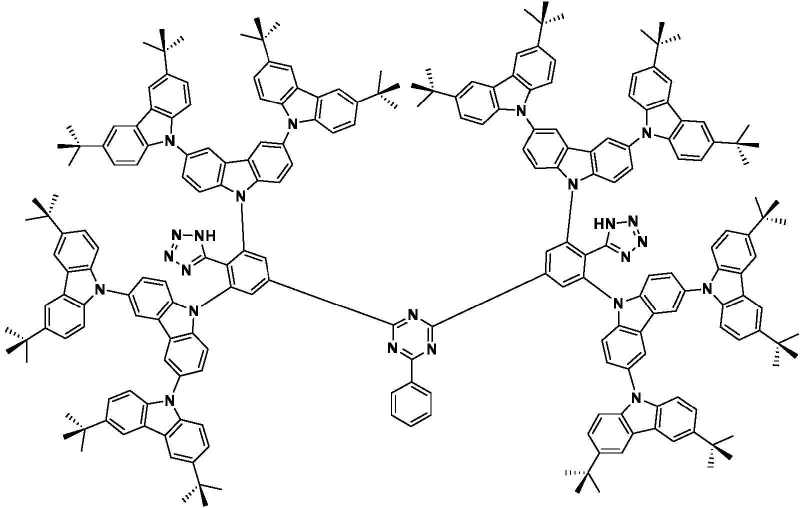 9',9ʺʺ,9‴ʺʺ,9‴‴ʺʺ-((6-phenyl-1,3,5-triazine-2,4-diyl)bis(2-(1H-tetrazol-5-yl)benzene-5,1,3-triyl))tetrakis(3,3",6,6"-tetra-tert-butyl-9'H-9,3':6',9"-tercarbazole) (tBuCz4m8NTRZ) |
| VIII | 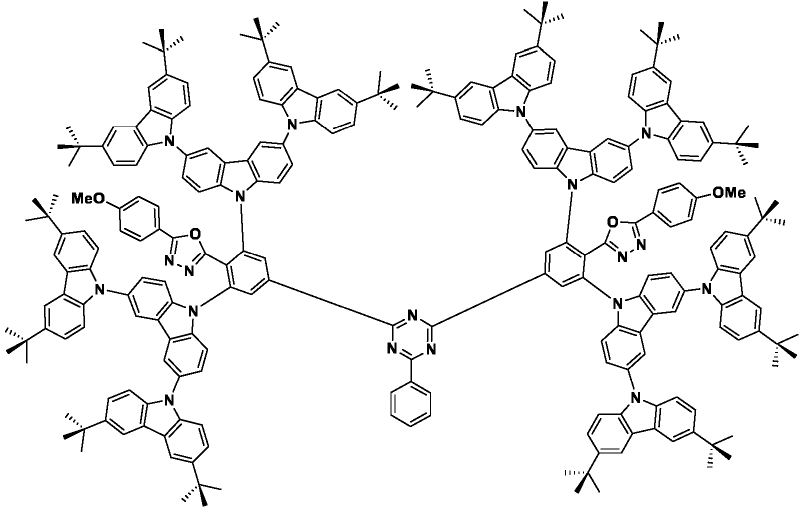 5,5'-((6-phenyl-1,3,5-triazine-2,4-diyl)bis(2,6-bis(3,3",6,6"-tetra-tert-butyl-9'H-[9,3':6',9"-tercarbazol]-9'-yl)-4,1-phenylene))bis(2-(4-methoxyphenyl)-1,3,4-oxadiazole) (tBuCz4mOXDOMeTRZ) |
| IX | 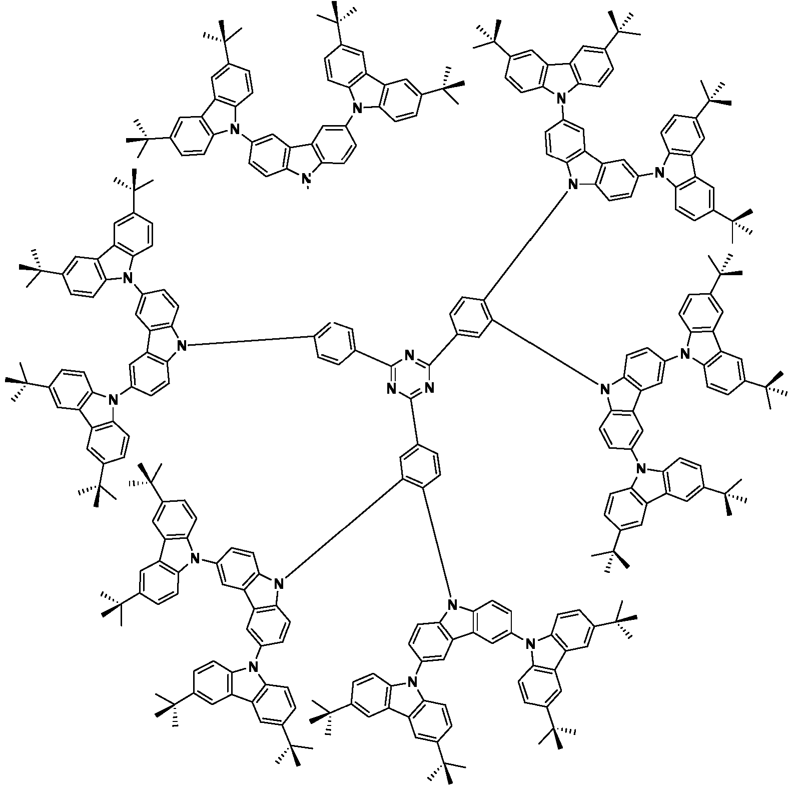 2,4,6-tris(3,4-bis(3,3",6,6"-tetra-tert-butyl-9'H-[9,3':6',9"-tercarbazol]-9'-yl)phenyl)-1,3,5-triazine (tBuCz3m3pTRZ) |
| X | 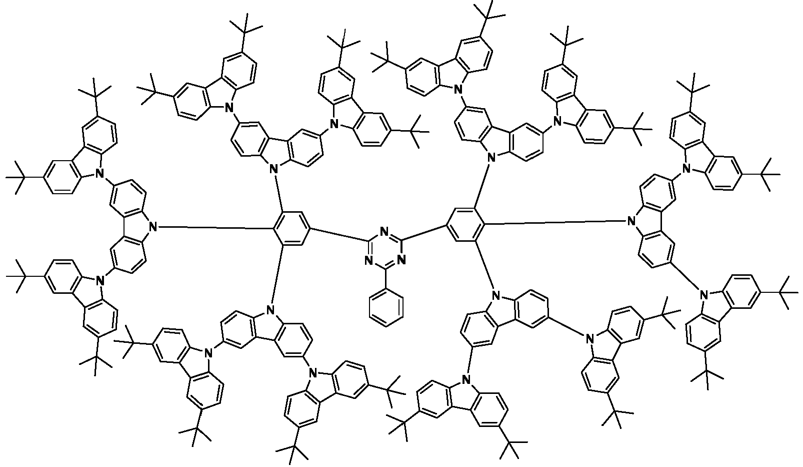 tBuCz4m2pTRZ |
| XI | 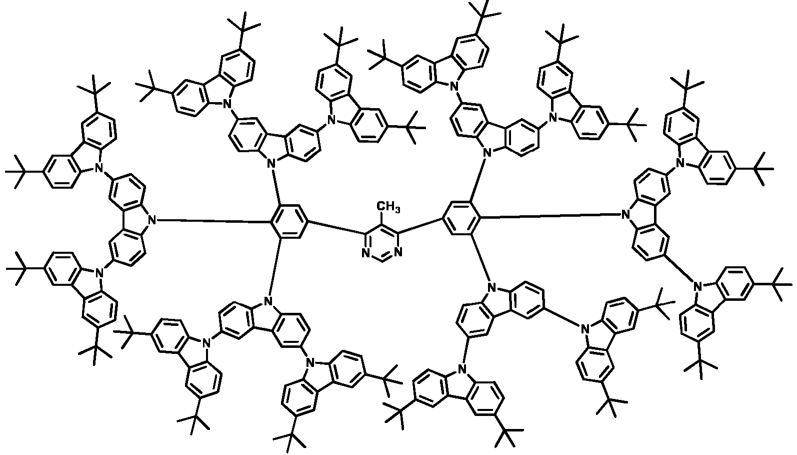 tBuCz4m2pPI |
| XIII | 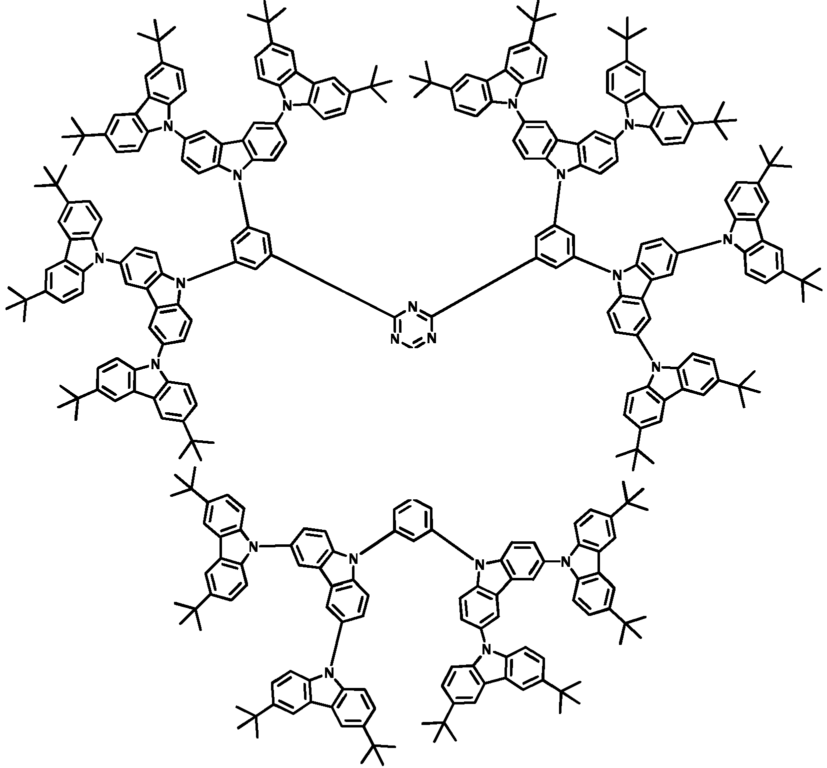 |
| XIV | 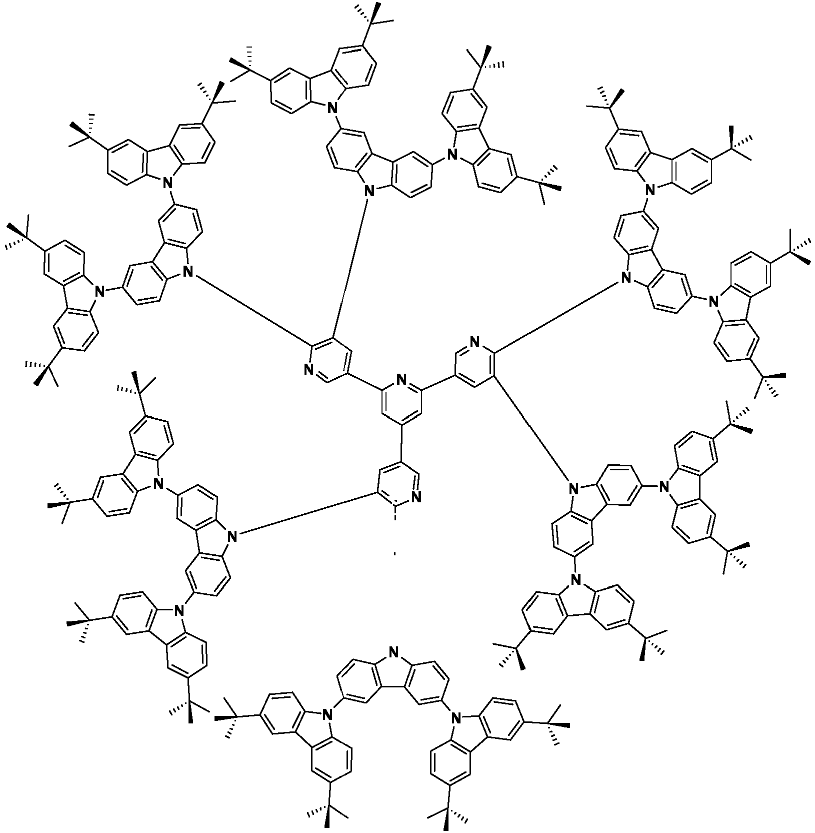 |
| XV | 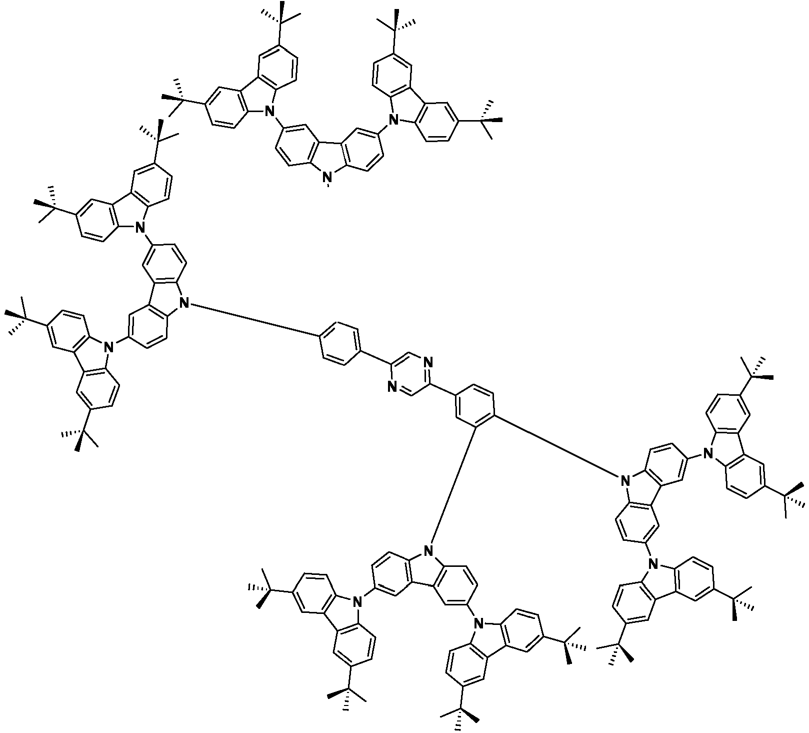 |
| XVI | 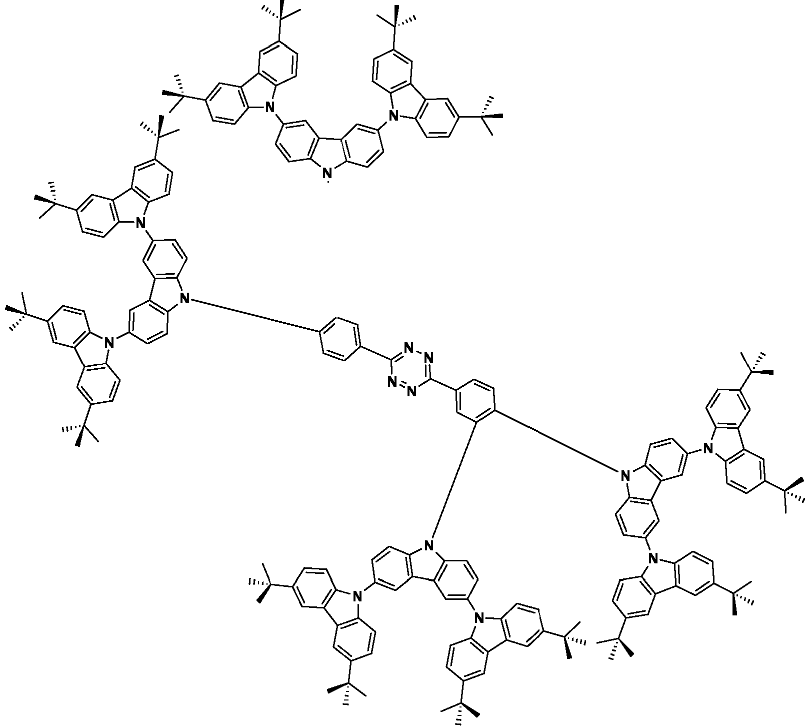 |

The dendrimers of the invention can be prepared by means known to the skilled person. Examples of preparation of dendrimers of the invention are described below with reference to specific embodiments.

In another aspect, the invention provides an electroluminescent device such as an OLED which comprises one or more compounds of the first aspect of the invention as emitter material. The electroluminescent device comprises an emissive layer (also referred to as light emitting layer) and the emissive layer comprises one or more compounds of the first aspect of the invention. The emissive layer may comprise other materials such as host materials. In one embodiment, the emissive layer contains is non-doped, i.e. it contains no host materials. In this embodiment, the emissive layer can consist essentially of one or more compounds of the first aspect of the invention.

In another aspect, the invention provides a method of preparing an electroluminescent device such as an OLED comprising depositing an emissive layer on a substrate using a solution processing technique, wherein said emissive layer comprises one or more compounds according to the first aspect of the invention.

The basic structure of an electroluminescent device such as an OLED is a thin film of organic material sandwiched between two electrodes. The electroluminescent device may be an OLED which comprises an anode, a cathode with the emissive layer situated between the anode and the cathode. For bottom emitting OLEDs, the anode, which is commonly Indium tin oxide (ITO) rests on top of a support such as glass but, metal foils and plastic substrates (such as polyethylene terephthalate (PET) or polyethylene naphthalate (PEN)) can be used. Poly(3,4-ethylenedixoythiophene):polystyrene sulfonate (PEDOT:PSS) is often deposited on top of the ITO anode as the hole injection layer. The next layer is an emissive layer and contains the emitter material which can be doped in a host material. The cathode is the final layer. The cathode is reflective and is made of metal such as aluminum, gold or silver, often combined with a very thin layer of lithium fluoride (LiF) or calcium to enhance the electron injection. The thickness of the organic layers in the basic structure is unusually between 100 and 150 nm. For bottom emitting OLEDs, the light emitting layer is deposited on the hole injection layer and then, typically, exciton blocking layer, electron transporting layer, electron injection layer and cathode are deposited subsequently. For top emitting OLEDs, the light emitting layer is deposited on electron transporting layer, and then, typically, the hole transporting layer, hole injection layer, and anode are deposited subsequently.

In the method of the invention, the substrate may be a hole injection layer or an electron transporting layer. Solution processing techniques are well known in the art and include inkjet printing, spin-coating, spray-coating, screen printing, dip-coating and slot-casting.

In the method of the invention, a solution comprising: one or more compounds of the invention; optionally, a host material; and, optionally, a solvent, is deposited on the substrate. The host material can be tris(4-carbazoyl-9-ylphenyl)amine, for example. The solvent can be chlorobenzene, chloroform, toluene, or tetrahydrofuran (THF), for example. The solution forms a film on the substrate. If the solution contains a solvent, the solvent is evaporated from the film. When the solution consists essentially of one or more compounds of the invention and a solvent, then the solvent is evaporated from the film to form an emissive layer consisting essentially of the one or more compounds of the invention. When the solution consists essentially of one or more compounds of the invention, a host material and a solvent, then the solvent is evaporated from the film to form an emissive layer consisting essentially of the one or more compounds of the invention and the host material.

The colour of the emitted light is a function of the energy difference between the highest occupied molecular orbital (HOMO) and the lowest unoccupied molecular orbital (LUMO) levels of the electroluminescent molecule. Compounds of the invention can be useful for emitting green and orange light, i.e. from about 500 nm to 580 nm.

The following definitions apply herein, unless a context dictates to the contrary.

The term "comprising" is intended also to encompass as alternative embodiments "consisting essentially of" and "consisting of." "Consisting essentially of" permits the inclusion of substances that do not materially affect the basic and novel characteristics of the composition under consideration.

By halo is meant chloro, fluoro, bromo or iodo.

By ester is meant a -OC(O)R substituent where R is selected from alkyl, aryl, aralkyl optionally substituted.

By haloalkyl is meant an alkyl group substituted with one or more halo substituents.

By sulfonyl is meant -S(=O)₂-R where R is a substituent such a hydrogen or an alkyl group or an aryl group.

By carbonyl is meant -C(O)R where R is a substituent such a hydrogen or an alkyl group or an aryl group.

By alkyl is meant a saturated hydrocarbyl radical, which may be straight-chain or branched. Typically, alkyl groups will comprise from 1 to 25 carbon atoms, more usually 1 to 10 carbon atoms, more usually still 1 to 6 carbon atoms. Thus, for example, "alkyl" can mean methyl, ethyl, propyl, isopropyl, butyl, isobutyl, tert-butyl, pentyl or hexyl.

By cycloalkyl is meant a saturated monocyclic or polycyclic hydrocarbyl radical. Typically cycloalkyl groups will comprise from 3 to 12 carbon atoms. Cycloalkyl groups include polycycloalkyl groups having a bicyclic, tricyclic, tetracyclic, pentacyclic or heptacyclic structure. The term cycloalkyl is further exemplified by radicals such as cyclopropyl, cyclobutyl, cyclopentyl, cyclohexyl, cycloheptyl, norbornyl, bicyclobutyl, bicyclohexyl, bicycloheptyl, bicyclooctyl, bicyclononyl, tricyclobutyl, tetracyclopentyl, pentacyclopentyl, heptocyclohexyl, the radical formed by abstraction of a hydrogen from prismane, and adamantyl. Adamantyl is considered to be a tricyclic polycyclalkyl group.

By alkoxy is meant -OR where R is an alkyl group.

By aryl is meant a radical of C6-C12 aromatic group, i.e. where the aromatic group has had a hydrogen abstracted. Aryl includes monocyclic and bicyclic aromatic groups. Examples include phenyl and naphthyl. Heteroaryl moieties are a subset of aryl moieties that comprise one or more heteroatoms (that may be the same or different), such as oxygen, nitrogen or sulphur, in place of one or more carbon atoms. Examples of suitable heteroaryl groups include thienyl, furanyl, pyrrolyl, pyridinyl, oxazolyl, thiazolyl, imidazolyl, pyrazolyl, isoxazolyl, isothiazolyl, oxadiazolyl, triazolyl, tetrazolyl, thiadiazolyl etc. Preferred heteroaryl groups include oxadiazolyl and tetrazoyl.

By aryloxy is meant -OR, where R is aryl.

By heteroarylaryloxy is meant -OR, where R is heteroaryl.

By silyl is meant is -SiR₃, where each R is the same or different and is a substituent such as a hydrogen or an alkyl group or an aryl group.

Where an alkyl, cycloalkyl, alkoxy, aryl, aryloxy, heteroaryl, heteroarloxy group is optionally substituted, this may be, unless a context expressly dictates otherwise, with one or more substituents independently selected from halo, ester, nitro, cyano, haloalkyl, sulfonyl, carbonyl group.

The invention may be further understood with reference to the following non-limiting numbered aspects and embodiments:
Aspect 1. A compound of formula (I),

(Ar₁)ₘ-(Ar₂)-(X)ₙ (I)

wherein:
Ar₂ is a pyridine, pyrimidine, pyrazine, pyridazine, triazine or tetrazine ring group;
m is from 1 to 5;
n is 0, 1 or 2;
each Ar₁ is independently selected from the following: 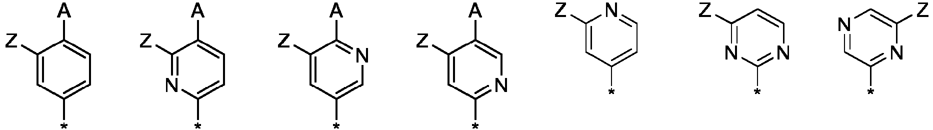 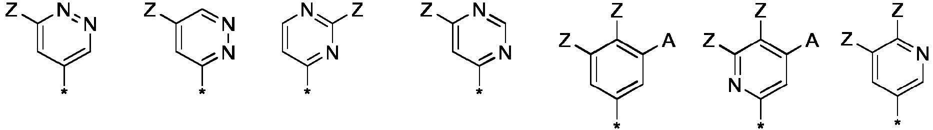 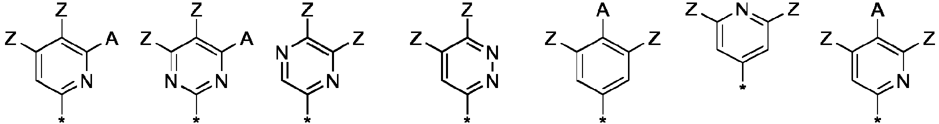 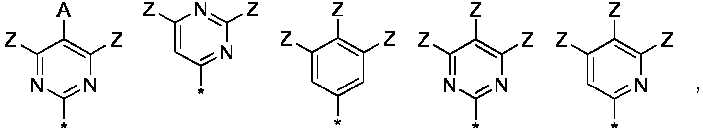
wherein each Z is independently selected from Z2 to Z4: 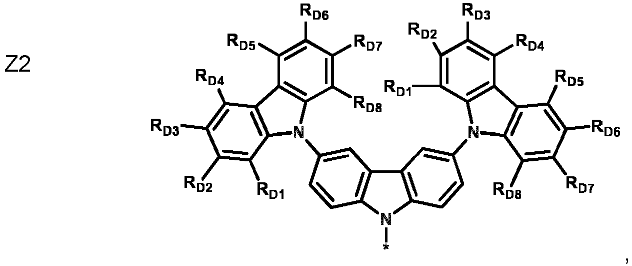 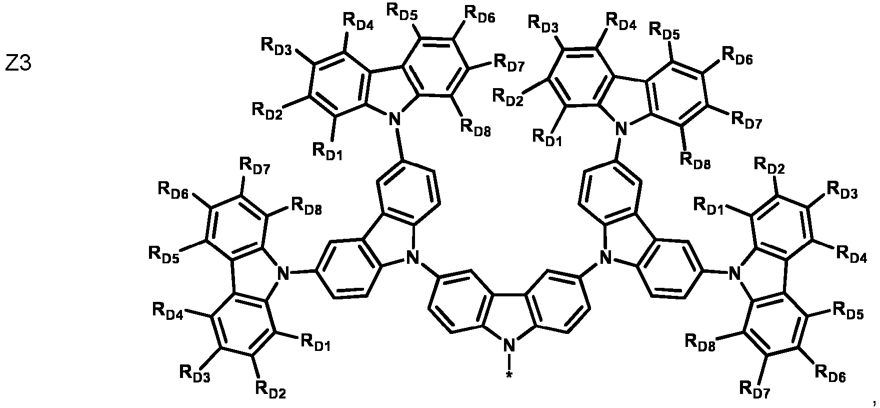 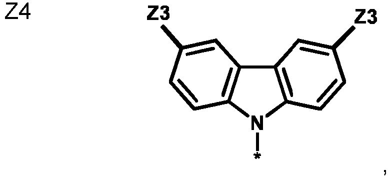
wherein R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl,
wherein R_{D2}, R_{D3}, R_{D6} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q,
wherein Q is selected from the following: 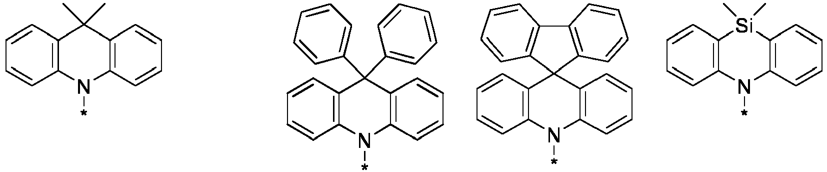 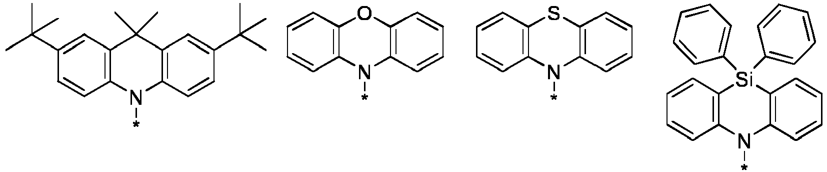 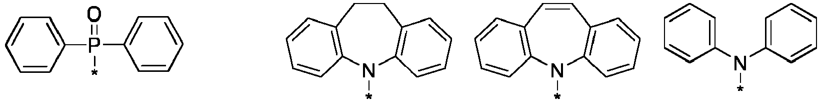 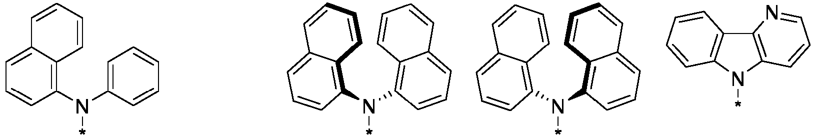 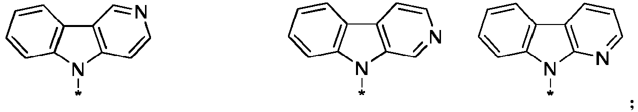
wherein A is selected from hydrogen, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy and silyl;
wherein each X is independently selected from substituted or unsubstituted alkyl, substituted or unsubstituted cycloalkyl and the following: 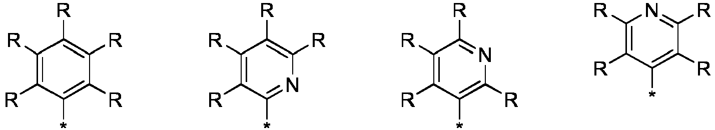 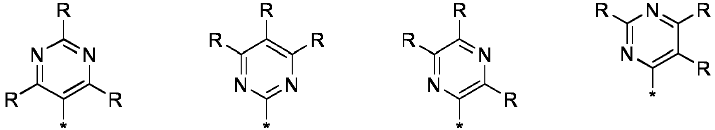 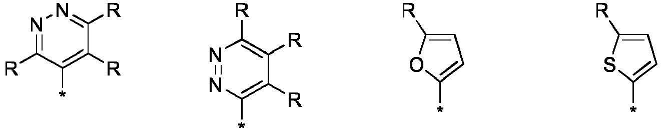 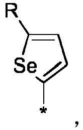
wherein each R is independently selected from the group consisting of hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and diphenylphosphine oxide;
wherein * represents a point of attachment; and
wherein for Z2, each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q.

In some embodiments of Aspect 1, m is two or more and the compound contains at least two different Ar₁ groups.

In some embodiments of Aspect 1, m is two or more and each of the Ar₁ groups is the same.

In Aspect 1, for Z2, each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In some embodiments, each of R_{D3} and R_{D6} is independently a substituted or unsubstituted alkyl (more preferably C1-C6 alkyl, even more preferably tertiary butyl). In some embodiments, each of R_{D1}, R_{D2}, R_{D4}, R_{D5}, R_{D7} and R_{D8} is hydrogen.

In some embodiments of Aspect 1, for Z3, each of each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In some embodiments, each of R_{D3} and R_{D6} is independently a substituted or unsubstituted alkyl (more preferably C1-C6 alkyl, even more preferably tertiary butyl). In some embodiments, each of R_{D1}, R_{D2}, R_{D4}, R_{D5}, R_{D7} and R_{D8} is hydrogen.

In some embodiments of Aspect 1, for Z4, each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q. In some embodiments, each of R_{D3} and R_{D6} is independently a substituted or unsubstituted alkyl (more preferably C1-C6 alkyl, even more preferably tertiary butyl). In some embodiments, each of R_{D1}, R_{D2}, R_{D4}, R_{D5}, R_{D7} and R_{D8} is hydrogen.

In some embodiments of Aspect 1,when Ar₁ is any of the following structures:

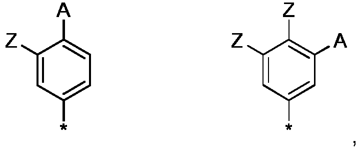

A is selected from hydrogen, halo, ester, nitro, cyano, haloalkyl, sulfonyl, carbonyl, substituted or unsubstituted alkyl, substituted or unsubstituted alkoxy, substituted or unsubstituted aryloxy, substituted or unsubstituted heteroaryl, substituted or unsubstituted heteroaryloxy and silyl.

In some embodiments of Aspect 1, when Ar₁ is any of the following structures:

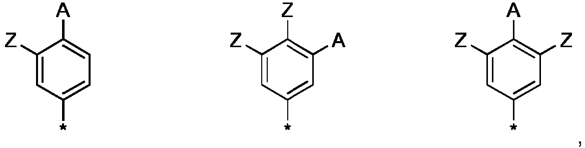

A is selected from hydrogen, halo, ester, nitro, cyano, haloalkyl, sulfonyl, carbonyl, substituted or unsubstituted alkyl, substituted or unsubstituted alkoxy, substituted or unsubstituted aryloxy, substituted or unsubstituted heteroaryl, substituted or unsubstituted heteroaryloxy and silyl.

In some embodiments of Aspect 1, A is selected from hydrogen, CN, tetrazole, or a substituted diazole.

In some embodiments of Aspect 1, each Z is independently selected from Z2.

In some embodiments of Aspect 1, the number of Z in formula (I) is 2 or more and each Z is the same.

In some embodiments of Aspect 1, the number of Z in formula (I) is 2 or more and the compound contains at least two different Z dendrons.

In some embodiments of Aspect 1, the number of Z in formula (I) is from 2 to 6.

In some embodiments of Aspect 1, m is 2 or 3, and, preferably, n is 0 or 1.

In some embodiments of Aspect 1, the number of Z dendrons in formula (I) is from two to four, preferably 3 or 4, more preferably 4.

In some embodiments of Aspect 1, Ar₂ is a triazine or a tetrazine ring group. In some embodiments, each Ar₁ is independently selected from:

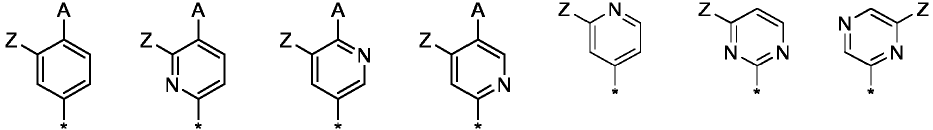

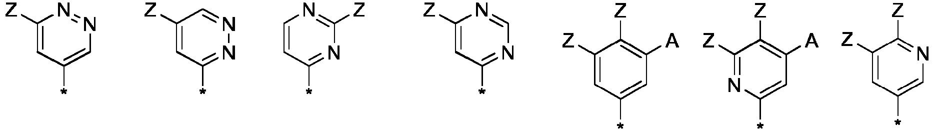

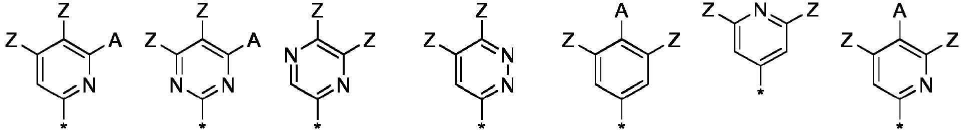

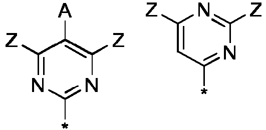

In some embodiments, and each Ar₁ is independently selected from:

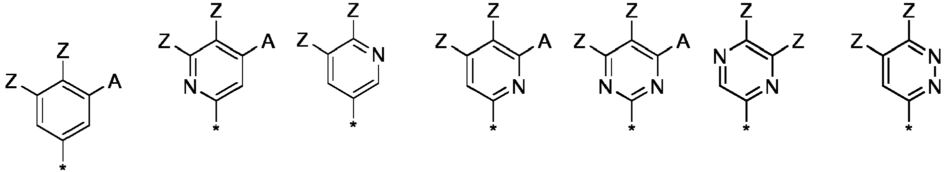

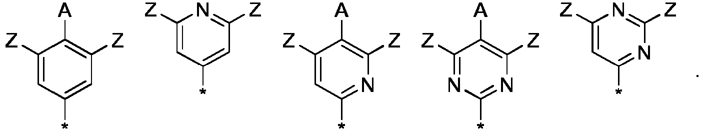

In some embodiments of Aspect 1, the number of Z dendrons in formula (I) is 5 or 6, preferably 6.

In some embodiments of Aspect 1, Ar₂ is a pyridine, pyrimidine, pyrazine, or pyridazine ring group. In some embodiments, each Ar₁ is independently selected from:

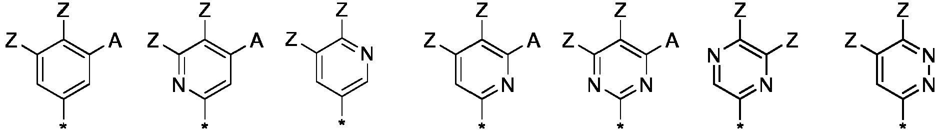

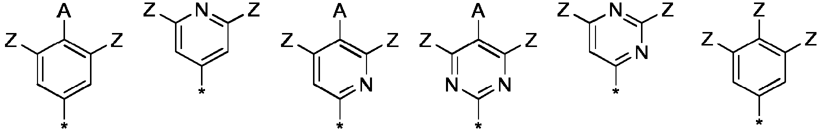

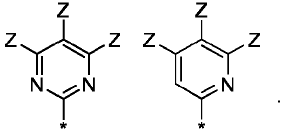

In some embodiments, m is 2, and each Ar₁ is independently selected from:

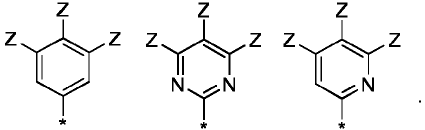

Aspect 2. An organic light emitting device comprising an emissive layer, wherein the emissive layer comprises one or more compounds according to Aspect 1 or Table 1.

In some embodiments of Aspect 1, the emissive layer consists essentially of one or more compounds according to Aspect 1 or Table 1.

Aspect 3. A method of preparing an organic light emitting device comprising depositing an emissive layer on a substrate using a solution processing technique, wherein said emissive layer comprises one or more compounds according to Aspect 1 or Table 1.

The invention is further described by way of the following non-limiting examples, and with reference to the following figures wherein:

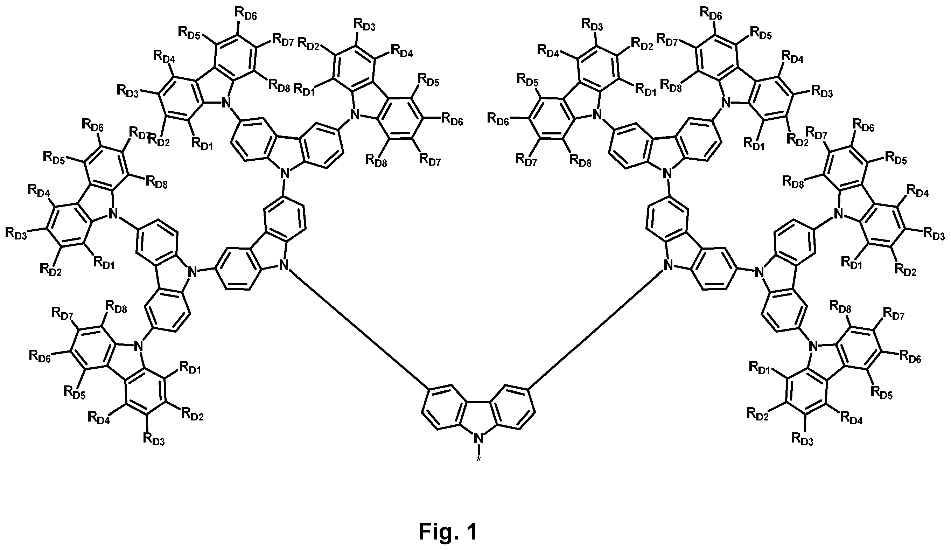

Figure 1 gives the structure of the dendron Z4.

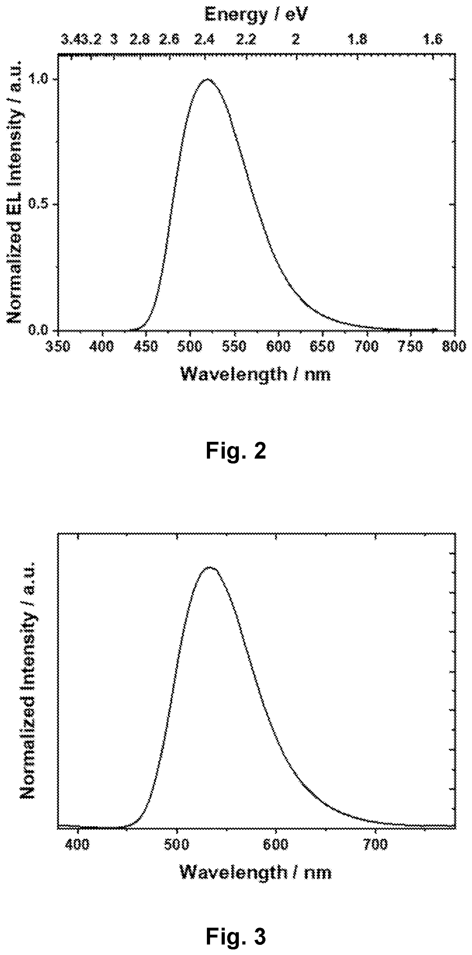

Figure 2 shows electroluminescence spectrum of Device 1 (100 wt% tBuCz2mTRZ).
Figure 3 shows electroluminescence spectrum of Device 2 (100 wt% tBuCz3mTRZ).

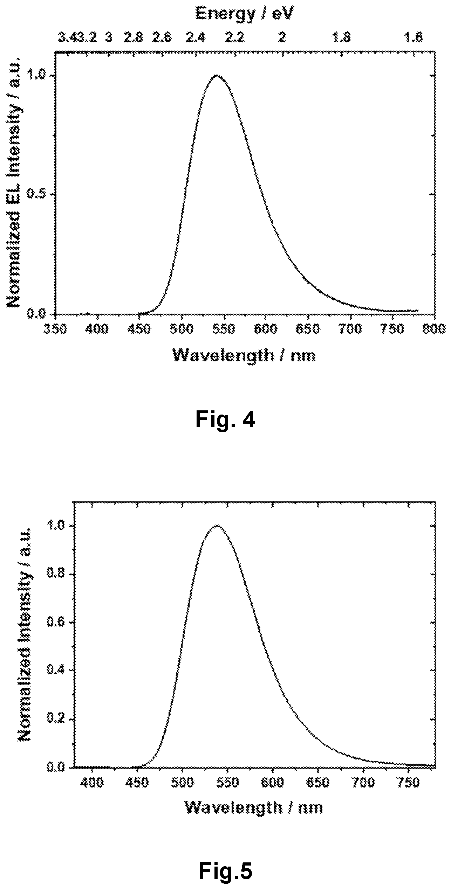

Figure 4 shows electroluminescence spectrum of Device 3 (100 wt% tBuCz2m2pTRZ).
Figure 5 shows electroluminescence spectrum of Device 4 (100 wt% tBuCz4mTRZ).

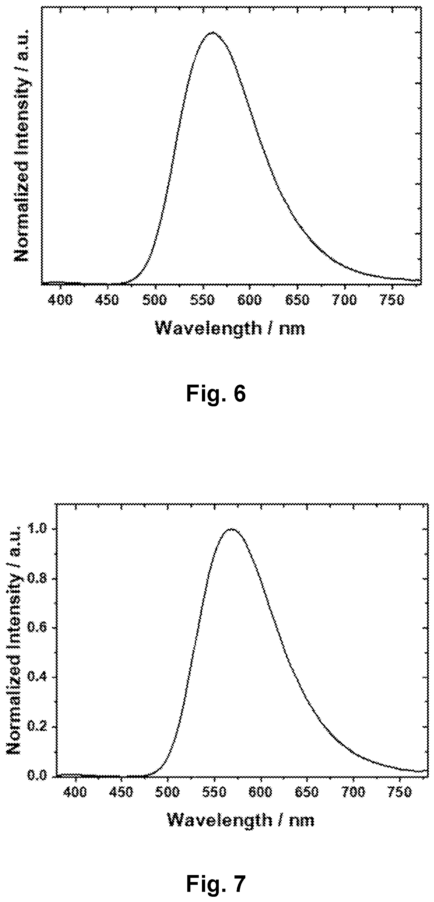

Figure 6 shows electroluminescence spectrum of Device 5 (10 wt% tBuCz3m3pTRZ in TCTA).
Figure 7 shows electroluminescence spectrum of Device 6 (100 wt% tBuCz3m3pTRZ).

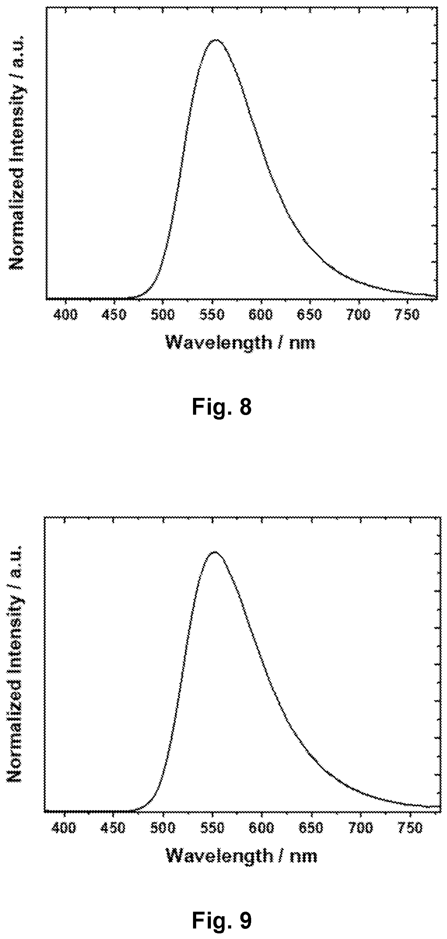

Figure 8 shows electroluminescence spectrum of Device 7 (10 wt% tBuCz4m2pTRZ in TCTA).
Figure 9 shows electroluminescence spectrum of Device 8 (100 wt% tBuCz4m2pTRZ).

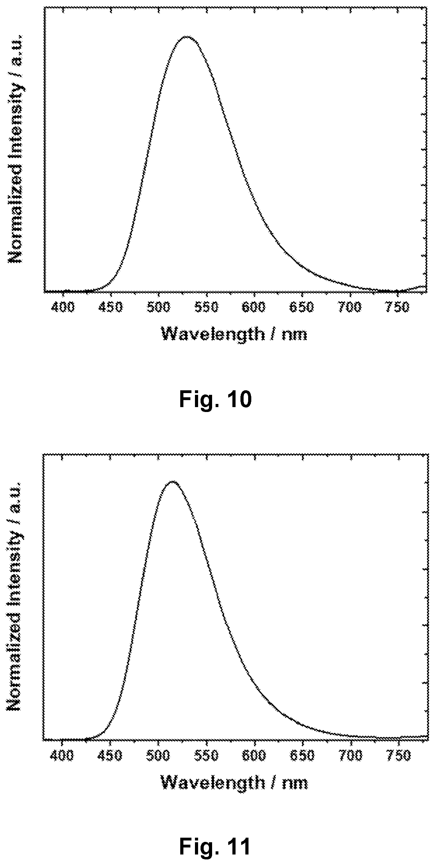

Figure 10 shows electroluminescence spectrum of Device 9 (10 wt% tBuCz4m2pPI in TCTA).
Figure 11 shows electroluminescence spectrum of Device 10 (100 wt% tBuCz4m2pPI).

### EXAMPLES

Compounds of the invention were synthesised as detailed below. The structure and names of the compounds are given in Table 1.

### Synthesis of compound I

Under nitrogen, a mixture of 3,3",6,6"-tetrakis(tert-butyl-9'H-9,3':6',9"-tercarbazole) (794 mg, 1.1 mmol, 2.2 equiv.), 2,4-bis(3-fluorophenyl)-6-phenyl-1,3,5-triazine (0.5 mmol, 1 equiv.) and cesium carbonate (652 mg, 2 mmol, 4 equiv.) in dry DMF (20 ml) was reflux for 24 h. After cooling to room temperature, the reaction was extracted with chloroform and washed with water (3 × 30 mL). The organic phase was dried over Na2SO4 and concentrated under reduced pressure. The crude product was purified by column chromatography on silica gel using 1:20 dichloromethane/hexane as eluent and then further purification was performed with preparative GPC column using THF as eluent to afford a yellow solid. Yield: 16.9%. Rf: 0.4 (Hexane: DCM=2:1). 1H NMR (400 MHz, CDCl3) δ (ppm): 9.17 (s, 2H), 9.01 (d, J=7.7Hz, 2H), 8.80 (d, J=7.7Hz, 2H), 8.31 (s, 4H), 8.17 (s, 8H), 8.00 (d, J=7.7Hz, 2H), 7.94 (t, J=7.7Hz, 2H), 7.71 (d, J=7.7Hz, 4H), 7.67 (d, J=7.7Hz, 5H), 7.58 (t, J=7.7Hz, 2H), 7.46 (d, J=7.7Hz, 8H), 7.38 (d, J=7.7Hz, 8H), 1.47 (s, 72H). 13C NMR (100 MHz, CDCl3) δ (ppm): 171.22, 142.63, 140.53, 140.14, 138.59, 137.99, 135.61, 131.48, 131.16, 130.78, 129.18, 128.89, 128.78, 127.85, 126.17, 124.14, 123.61, 123.17, 119.41, 116.27, 111.15, 109.10, 34.76, 32.07. MALDI-TOF-MS: Calculated: 1749.41, Found: 1749.28. Anal. Calcd. For C125H121N9 (%): C, 85.82; H, 6.97; N, 7.21; Found: C, 85.71; H, 7.09; N, 7.20.

### Synthesis of compound II

Under nitrogen, a mixture of 3,3",6,6"-tetrakis(tert-butyl-9'H-9,3':6',9"-tercarbazole) (794 mg, 1.1 mmol, 3.3 equiv.), 2,4,6-tris(3-fluorophenyl)-1,3,5-triazine (134 mg, 0.37 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na2SO4 and concentrated under reduced pressure. The crude product was purified by column chromatography on silica gel using 1:20 dichloromethane/hexane as eluent and then further purification was performed with preparative GPC column using THF as eluent to afford a yellow solid. Yield: 57.2%. Rf: 0.3 (Hexane: DCM=2:1). 1H NMR (400 MHz, CDCl3) δ 9.22 (t, J = 1.9 Hz, 3H), 9.00 (dt, J = 7.8, 1.4 Hz, 3H), 8.36 - 8.27 (m, 6H), 8.23 - 8.12 (m, 12H), 8.04 (ddd, J = 7.9, 2.2, 1.3 Hz, 3H), 7.97 (t, J = 7.8 Hz, 3H), 7.78 - 7.71 (m, 6H), 7.68 (dd, J = 8.7, 2.0 Hz, 6H), 7.47 (dd, J = 8.7, 1.9 Hz, 12H), 7.38 (dd, J = 8.5, 0.7 Hz, 12H), 1.48 (s, 108H).

### Synthesis of compound III

Under nitrogen, a mixture of 3,3",6,6"-tetrakis(tert-butyl-9'H-9,3':6',9"-tercarbazole) (794 mg, 1.1 mmol, 4.4 equiv.), 2,4-bis(3,4-difluorophenyl)-6-phenyl-1,3,5-triazine (95 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford a yellow solid (654 mg). Yield: 82%. Rf: 0.4 (Hexane: DCM=2:1). 1H NMR (400 MHz, CDCl3) δ (ppm): 9.70 (s, 2H), 9.49 (d, J=8.2Hz, 2H), 8.99 (d, J=7.7Hz, 2H), 8.51 (d, J=8.2Hz, 2H), 8.01 (s, 16H), 8.03 (d, J=11.2Hz, 8H), 7.72 (q, J=7.6Hz, 3H), 7.45 (t, J=8.6Hz, 6H), 7.41 (s, 3H), 7.31 (t, J=8.0Hz, 9H), 7.07 (brs, 30H), 1.33 (s, 144H). 13C NMR (100 MHz, CDCl3) δ (ppm): 142.51, 139.78, 139.20, 139.01, 131.57, 131.45, 125.26, 124.47, 124.32, 123.66, 123.04, 118.91, 116.11, 111.20, 108.70, 34.62, 31.98, 30.96. MALDI-TOF-MS: Calculated: 3189.44, Found: 3189.43. Anal. Calcd. For C229H227N15 (%): C, 86.24; H, 7.17; N, 6.59; Found: C, 86.21; H, 7.19; N, 6.60.

### Synthesis of compound IV

Under nitrogen, a mixture of 3,3",6,6"-tetrakis(tert-butyl-9'H-9,3':6',9"-tercarbazole) (794 mg, 1.1 mmol, 4.4 equiv.), 2,4-bis(3,5-difluorophenyl)-6-phenyl-1,3,5-triazine (95 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:10 dichloromethane/hexane as eluent and then further purification was performed with preparative GPC column using THF as eluent to afford a yellow solid (360 mg). Yield: 45%. Rf: 0.35 (Hexane: DCM=2:1 on silica plate). 1H NMR (400 MHz, Chloroform-d): δ 9.35 (d, J = 2.0 Hz, 4H), 8.93 - 8.87 (m, 2H), 8.44 (t, J = 2.0 Hz, 2H), 8.35 - 8.28 (m, 8H), 8.16 (dd, J = 1.9, 0.7 Hz, 16H), 7.96 - 7.87 (m, 8H), 7.72 (dd, J = 8.7, 2.0 Hz, 9H), 7.69 - 7.61 (m, 2H), 7.41 (dd, J = 8.7, 1.9 Hz, 16H), 7.34 (dd, J = 8.6, 0.6 Hz, 16H), 1.44 (s, 144H). 13C NMR (101 MHz, CDCl3): δ 142.71, 140.09, 139.97, 131.77, 126.37, 124.51, 123.62, 123.21, 119.58, 116.29, 111.00, 108.99, 77.35, 77.04, 76.72, 34.72, 32.02. MALDI-TOF-MS: Calculated: 3189.44, Found: 3189.43. Anal. Calcd. For C229H227N15 (%): C, 86.24; H, 7.17; N, 6.59; Found: C, 86.11; H, 7.04; N, 6.57.

### Synthesis of compound V

Under nitrogen, a mixture of 3,3",6,6"-tetrakis(tert-butyl-9'H-9,3':6',9"-tercarbazole) (794 mg, 1.1 mmol, 4.4 equiv.), 2,4-bis(3,4-difluorophenyl)-6-phenyl-1,3,5-triazine (96 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford an orange solid (630 mg). Yield: 79%. Rf: 0.6 (Hexane: DCM=1:1). 1H NMR (400 MHz, CDCl3) δ 10.30 (d, J = 2.1 Hz, 2H), 9.78 (d, J = 2.0 Hz, 2H), 9.42 (d, J = 3.1 Hz, 4H), 8.83 (d, J = 8.0 Hz, 4H), 8.39 (s, 4H), 8.22 - 8.15 (m, 10H), 8.15 - 8.03 (m, 20H), 7.98 (d, J = 2.0 Hz, 4H), 7.75 (d, J = 3.8 Hz, 12H), 7.64 (t, J = 7.5 Hz, 6H), 7.57 (d, J = 8.7 Hz, 4H), 7.45 (qd, J = 8.6, 1.9 Hz, 20H), 7.37 (dd, J = 8.4, 6.5 Hz, 10H), 7.27 (d, J = 1.8 Hz, 4H), 7.09 (s, 30H), 1.36 (s, 144H).

### Synthesis of compound VI

Under nitrogen, a mixture of 3,3",6,6"-tetrakis(tert-butyl-9'H-9,3':6',9"-tercarbazole) (794 mg, 1.1 mmol, 4.4 equiv.), 4,4'-(6-phenyl-1,3,5-triazine-2,4-diyl)bis(2,6-difluorobenzonitrile) (108 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford a red solid (65 %). 1H NMR (400 MHz, CDCl3) δ 9.30 (s, 4H), 8.52 (d, J = 7.6 Hz, 4H), 8.40 - 8.34 (m, 8H), 8.20 (dd, J = 1.9, 0.7 Hz, 16H), 7.80 (dd, J = 8.7, 2.0 Hz, 8H), 7.72 (p, J = 7.3 Hz, 9H), 7.51 (dd, J = 8.7, 1.9 Hz, 16H), 7.45 (dd, J = 8.6, 0.6 Hz, 16H), 1.50 (s, 144H).

### Synthesis of compound VII

A Schlenk flask under nitrogen atmosphere was charged with 4,4'-(6-phenyl-1,3,5-triazine-2,4-diyl)bis(2,6-bis(3,3",6,6"-tetra-tert-butyl-9'H-[9,3':6',9"-tercarbazol]-9'-yl)benzonitrile) (0.44 mmol, 1.00 equiv.), ammonium chloride (0.28 g, 5.3 mmol, 12.00 equiv.), sodium azide (0.34 g, 5.3 mmol, 12.00 equiv.) and anhydrous DMF (15 mL). The reaction mixture was heated at 120 °C for 24 h. After cooling to room temperature, the reaction mixture was poured into a 1M HCI solution (15 mL) resulting in a white precipitate crashing out. The white precipitate was filtered and washed with deionized water (3 × 30 mL) to give the title compound as a yellow solid (80%). This crude product was thoroughly dried and then used for the next step without further purification. 1H NMR (400 MHz, CDCl3) δ 9.33 (s, 4H), 8.51 (d, J = 7.6 Hz, 4H), 8.25 (d, J = 2.0 Hz, 8H), 8.21 - 8.13 (m, 17H), 7.74 (d, J = 7.4 Hz, 4H), 7.69 (d, J = 7.8 Hz, 4H), 7.64 (dd, J = 8.7, 2.0 Hz, 8H), 7.57 (d, J = 8.6 Hz, 8H), 7.48 (dd, J = 8.7, 1.9 Hz, 12H), 7.38 (d, J = 8.6 Hz, 12H), 1.48 (s, 144H).

### Synthesis of compound VIII

A Schlenk flask under nitrogen atmosphere was charged with 9',9ʺʺ,9‴ʺʺ,9‴‴ʺʺ-((6-phenyl-1,3,5-triazine-2,4-diyl)bis(2-(1H-tetrazol-5-yl)benzene-5,1,3-triyl))tetrakis(3,3",6,6"-tetra-tert-butyl-9'H-9,3':6',9"-tercarbazole) (333 mg, 0.1 mmol, 1.00 equiv.) and anhydrous pyridine (5 mL). 4-methoxybenzoyl chloride (6.00 equiv.) was added dropwise. The reaction mixture was then stirred at 110 °C overnight for 24 h. After cooling to room temperature, the mixture was poured into a 1M HCI solution (50 mL). The mixture was then extracted with DCM (3 × 20 mL) and the combined organic layers were dried with anhydrous sodium sulphate and concentrated under reduced pressure. The resulting crude product was purified by column chromatography on silica gel using 1:20 dichloromethane/hexane as eluent and then further purification was performed with preparative GPC column using THF as eluent to afford an orange-red solid. Yield: 55%. Rf: 0.2 (Hexane: DCM=1:1). 1H NMR (400 MHz, CDCl3) δ 9.45 (s, 1H), 8.23 (dd, J = 1.9, 0.8 Hz, 2H), 8.18 - 8.15 (m, 4H), 8.14 (d, J = 2.0 Hz, 16H), 8.12 (d, J = 2.1 Hz, 18H), 8.09 (d, J = 2.0 Hz, 2H), 8.07 (d, J = 2.1 Hz, 2H), 7.82 - 7.74 (m, 1H), 7.71 (t, J = 7.8 Hz, 2H), 7.64 (dd, J = 4.1, 1.3 Hz, 4H), 7.42 (d, J = 8.8 Hz, 8H), 7.25 (d, J = 8.9 Hz, 2H), 7.05 - 6.98 (m, 22H), 6.97 (d, J = 9.0 Hz, 4H), 6.80 (d, J = 8.9 Hz, 1H), 3.92 (s, 6H), 1.47 (s, 144H).

### Synthesis of compound IX

Under nitrogen, a mixture of 3,3",6,6"-tetrakis(tert-butyl-9'H-9,3':6',9"-tercarbazole) (1.16 g, 1.6 mmol, 6.4 equiv.), 2,4,6-tris(3,4-difluorophenyl)-1,3,5-triazine (104 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford a yellow solid. Yield: 71%. Rf: 0.4 (Hexane: DCM=2:1). 1H NMR (400 MHz, CDCl3) δ 9.81 (d, J = 2.1 Hz, 4H), 9.55 (dd, J = 8.3, 2.0 Hz, 4H), 8.56 (d, J = 8.4 Hz, 4H), 8.18 - 7.93 (m, 40H), 7.51 (d, J = 8.6 Hz, 8H), 7.44 (d, J = 8.6 Hz, 8H), 7.34 (ddd, J = 10.8, 8.6, 2.0 Hz, 15H), 7.07 (s, 34H), 1.36 (s, 116H), 1.32 (s, 100H).

### Synthesis of compound X

Under nitrogen, a mixture of 3,3",6,6"-tetrakis(tert-butyl-9'H-9,3':6',9"-tercarbazole) (1.16 g, 1.6 mmol, 6.4 equiv.), 2-phenyl-4,6-bis(3,4,5-trifluorophenyl)-1,3,5-triazine (104 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford a yellow solid. Yield: 38%. Rf: 0.3 (Hexane: DCM=2:1). 1H NMR (400 MHz, CDCl3) δ 9.82 (s, 4H), 8.94 (d, J = 0.8 Hz, 4H), 8.08 (d, J = 17.4 Hz, 31H), 7.92 - 7.79 (m, 4H), 7.76 (d, J = 8.0 Hz, 8H), 7.63 (d, J = 1.8 Hz, 8H), 7.51 - 7.36 (m, 12H), 7.22 (d, J = 1.9 Hz, 8H), 7.17 - 7.04 (m, 8H), 6.99 (d, J = 8.6 Hz, 6H), 6.97 - 6.91 (m, 8H), 6.89 (dd, J = 7.6, 1.3 Hz, 8H), 6.65 (d, J = 8.6 Hz, 4H), 6.55 - 6.46 (m, 4H), 1.30 (s, 216H).

### Synthesis of compound XI

Under nitrogen, a mixture of 3,3",6,6"-tetrakis(tert-butyl-9'H-9,3':6',9"-tercarbazole) (1.16 g, 1.6 mmol, 6.4 equiv.), 5-methyl-4,6-bis(3,4,5-trifluorophenyl)pyrimidine (88.5 mg, 0.25 mmol, 1 equiv.) and caesium carbonate (652 mg, 2 mmol, 8 equiv.) in dry DMF (20 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:20 dichloromethane/hexane as eluent and then further purification was performed with preparative GPC column using THF as eluent to afford a yellow solid. Yield: 77 %. Rf: 0.5 (Hexane: DCM=2:1). 1H NMR (400 MHz, CDCl3) δ 9.74 (s, 2H), 8.90 (s, 4H), 8.15 - 8.00 (m, 33H), 7.85 (d, J = 2.0 Hz, 8H), 7.76 (d, J = 8.7 Hz, 8H), 7.44 (d, J = 8.8 Hz, 8H), 7.12 (dd, J = 8.7, 2.0 Hz, 30H), 6.94 (d, J = 8.6 Hz, 12H), 6.88 (dd, J = 8.7, 1.9 Hz, 8H), 1.35 (s, 128H), 1.29 (d, J = 2.2 Hz, 91H).

### Synthesis of compound XIII

Under nitrogen, a mixture of 3,3",6,6"-tetrakis(tert-butyl-9'H-9,3':6',9"-tercarbazole) (1.16 g, 1.6 mmol, 6.4 equiv.), 2,4,6-tris(3,5-difluorophenyl)-1,3,5-triazine (104 mg, 0.25 mmol, 1 equiv.) and caesium carbonate (1.47 g, 4.5 mmol, 18 equiv.) in dry DMF (30 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using hexane as eluent and then further purification was performed with preparative GPC column using THF as eluent to afford a yellow solid. Yield: 28%. Rf: 0.4 (Hexane: DCM=2:1). 1 H NMR (400 MHz, CDCl3) δ 9.39 - 8.94 (m, 8H), 8.27 (ddq, J = 13.4, 7.0, 2.2 Hz, 8H), 8.22 - 8.04 (m, 26H), 7.95 - 7.72 (m, 9H), 7.72 - 7.50 (m, 16H), 7.50 - 7.32 (m, 32H), 7.29 (s, 18H), 1.53 - 1.35 (m, 27H).

### Synthesis of compound XIV

Under nitrogen, a mixture of 3,3",6,6"-tetrakis(tert-butyl-9'H-9,3':6',9"-tercarbazole) (1.16 g, 1.6 mmol, 6.4 equiv.), 6'-(5,6-difluoropyridin-3-yl)-5,5",6,6"-tetrafluoro-3,2':4',3"-terpyridine (105 mg, 0.25 mmol, 1 equiv.) and caesium carbonate (1.47 g, 4.5 mmol, 18 equiv.) in dry DMF (30 ml) was reflux for 48 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using 1:30 dichloromethane/hexane as eluent and then further purification was performed with preparative GPC column using THF as eluent to afford a green solid. Yield: 49%. Rf: 0.3 (Hexane: DCM=4:1). 1H NMR (400 MHz, CDCl3) δ 9.72 (s, 4H), 9.37 (s, 4H), 8.29 (d, J = 8.5 Hz, 4H), 8.15 - 7.89 (m, 40H), 7.58 (d, J = 8.6 Hz, 8H), 7.40 (d, J = 8.6 Hz, 8H), 7.33 - 7.19 (m, J = 10.8, 8.6, 2.0 Hz, 14H), 7.10 (s, 34H), 1.36 - 1.32 (m, 216H).

### Synthesis of compound XV

Under nitrogen, a mixture of 3,3",6,6"-tetrakis(tert-butyl-9'H-9,3':6',9"-tercarbazole) (1.60 g, 2.2 mmol, 4.4 equiv.), 2,5-bis(3,4-difluorophenyl)pyrazine (152 mg, 0.5 mmol, 1 equiv.) and caesium carbonate (1.96 g, 6 mmol, 12 equiv.) in dry DMF (40 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using hexane as eluent and then further purification was performed with preparative GPC column using THF as eluent to afford a green solid. Yield: 72%. R*f*: 0.4 (Hexane: DCM=3:1). 1H NMR (400 MHz, CDCl3) δ 9.82 (d, J = 1.5 Hz, 4H), 9.38 (d, J = 1.5 Hz, 4H), 8.36 - 8.27 (m, 16H), 8.21 (dd, J = 2.0, 0.6 Hz, 16H), 7.78 (dd, J = 8.7, 2.1 Hz, 8H), 7.50 (dd, J = 8.7, 1.9 Hz, 16H), 7.42 (dd, J = 8.6, 0.6 Hz, 4H), 1.50 (s, 144H).

### Synthesis of compound XVI

Under nitrogen, a mixture of 3,3",6,6"-tetrakis(tert-butyl-9'H-9,3':6',9"-tercarbazole) (1.60 g, 2.2 mmol, 4.4 equiv.), 3,6-bis(3,4-difluorophenyl)-1,2,4,5-tetrazine (151 mg, 0.5 mmol, 1 equiv.) and caesium carbonate (1.96 g, 6 mmol, 12 equiv.) in dry DMF (40 ml) was reflux for 24 h. After cooling to room temperature, the reaction was extracted with chloroform and washed with water (3 × 30 mL). The organic phase was dried over Na₂SO₄ and concentrated under reduced pressure. The crude product was purified by column chromatography on silica gel using hexane as eluent and then further purification was performed with preparative GPC column using THF as eluent to afford a yellow solid. Yield: 41%. Rf: 0.3 (Hexane: DCM=3:1). 1H NMR (400 MHz, CDCl3) δ 9.78 (s 4H), 9.29 (s, 4H), 8.32 - 8.21 (m, 30H), 7.71 (m, 8H), 7.44 (m, 16H), 7.32 (m, 16H), 1.39 (s, 144H).

### OLED Device Performance

Ten OLED devices were constructed to compare the properties of TADF compounds. Device 1 comprises compound I (TADF dendrimer **tBuCz2mTRZ**) as an emitting layer. Device 2 comprises compound II (TADF dendrimer **tBuCz3mTRZ**) as an emitting layer. Device 3 comprises compound III (TADF dendrimer **tBuCz2m2pTRZ**) as an emitting layer. Device 4 comprises compound IV (TADF dendrimer **tBuCz4mTRZ**) as an emitting layer. Device 5 comprises 10 wt % compound IX (TADF dendrimer **tBuCz3m3pTRZ**) into TCTA host as an emitting layer. Device 6 comprises compound IX (TADF dendrimer **tBuCz3m3pTRZ**) as an emitting layer. Device 7 comprises 10 wt % compound X (TADF dendrimer **tBuCz4m2pTRZ**) into TCTA host as an emitting layer. Device 8 comprises compound X (TADF dendrimer **tBuCz4m2pTRZ**) as an emitting layer. Device 9 comprises compound XI (10 wt % TADF dendrimer **tBuCz4m2pPI**) into TCTA host as an emitting layer. Device 10 comprises compound XI (TADF dendrimer **tBuCz4m2pPI**) as an emitting layer. The devices have the following layered structure: ITO/PEDOT: PSS (35 nm)/ emitting layer (40 nm)/TmPyPB (40 nm)/LiF (1 nm)/ Al (100 nm). TmPyPB is 1,3,5-Tri(m-pyridin-3-ylphenyl)benzene.

The OLED devices were fabricated using a solution-processing technique and bottomemitting architecture. A pre-patterned indium tin oxide (ITO) glass substrate with a sheet resistance of 15 Ω square⁻¹ was pre-cleaned carefully with detergent and deionized water and then exposed to UV-ozone for 15 min. PEDOT: PSS was spin-coated onto the clean ITO substrate as hole-injection layer, followed by thermal treatment at 120 °C for 30 minutes. Then, for the non-doped emissive layers, 10mg/mL of dendrimer in chlorobenzene solution was spin-coated onto the PEDOT:PSS layer to form a 35-65 nm thick emissive layer (EML) and annealed at 120 °C for 10 minutes to remove residual solvent before the transfer to the vacuum chamber with a base pressure of 4×10-4 Pa. For the doped emissive layers, TCTA, Tris(4-carbazoyl-9-ylphenyl)amine, is used as a host to disperse dendrimer emitters in the emissive layer. 10 wt% dendrimer was blended with TCTA and then dissolved into chlorobenzene to form a 10mg/mL solution. This was then spin-coated onto the PEDOT:PSS layer to form an emissive layer (EML) and annealed at 120 °C for 10 minutes to remove residual solvent before the transfer to the vacuum chamber with a base pressure of 4×10-4 Pa. A 40 nm-thick electrontransporting layer (ETL) of Tm3PyPB was then vacuum deposited onto the EML at a rate of 1 Å/s, which was controlled in situ using the quartz crystal monitors, followed by the deposition of an electron injection layer LiF and an Al cathode. The electron injection layer LiF was deposited at a rate of 0.1 Å/s while the Al cathode was deposited at a rate of 10 Å/s through the shadow mask defining the top electrode.

The green electroluminescence of each of devices 1, 2, 3, 4, 9 and 10 is demonstrated by the λ_{EL} and Commission Internationale de L'Éclairage (CIE) coordinates as shown in Table 2. The orange electroluminescence of each of devices 5, 6, 7 and 8 is demonstrated the λ_{EL} and Commission Internationale de L'Éclairage (CIE) coordinates as shown in Table 2.

**Table 2 - Summary of device performance**

| Device No. | Compou nd | λ_{EL} / nm | FWH M ^{a)} / nm | Vₒₙ ^{b)} *N* | CEmax c) / cd A⁻¹ | PEₘₐₓ^{d)} / lm W⁻¹ | EQEₘₐₓ^{e )} /% | CIE^{f)} (x,y) | Lumₘₐₓ^{g )} / cd m⁻² |
|---|---|---|---|---|---|---|---|---|---|
| **1** | I | 516 | 95 | 3.3 | 51.1 | 44.6 | 17.0 | 0.27, 0.53 | 973 |
| **2** | II | 532 | 90 | 3.4 | 51.7 | 43.9 | 15.2 | 0.33, 0.58 | 2927 |
| **3** | II | 540 | 97 | 3.1 | 98.8 | 91.3 | 28.7 | 0.37, 0.57 | 6029 |
| **4** | IV | 536 | 91 | 3.0 | 96.9 | 82.3 | 28.4 | 0.36, 0.58 | 1855 |
| **5** | IX-doped | 560 | 97 | 3.4 | 58.1 | 35.1 | 17.8 | 0.44, 0.54 | 2294 |
| **6** | IX | 568 | 98 | 3.2 | 31.3 | 25.9 | 10.2 | 0.47, 0.52 | 3444 |
| **7** | X-doped | 560 | 98 | 3 | 27.67 | 26.34 | 8.57 | 0.44,0.54 | 468 |
| **8** | X | 560 | 96 | 3.2 | 24.81 | 19.01 | 7.76 | 0.45,0.53 | 784 |
| **9** | XI-doped | 520 | 92 | 2.8 | 43.8 | 41.7 | 14.1 | 0.29, 0.55 | 2323 |
| **10** | XI | 508 | 85 | 3.3 | 53.8 | 44.5 | 18.2 | 0.25, 0.52 | 1547 |

| | | | | | | | | | |
|---|---|---|---|---|---|---|---|---|---|
| ^{a)} Full wavelength at highest maximum ^{b)} Turn-on voltage at the luminance of 1 cd m ⁻² ^{c)} CEₘₐₓ -Maximum current efficiency ^{d)} PEₘₐₓ -Maximum power efficiency ^{e)} EQEₘₐₓ-Maximum external quantum efficiency. ^{f)} CIE -The Commission Internationale de L'Eclairage coordinates at 1 mA/cm². ^{g)} Lumₘₐₓ - Maximum Luminance | | | | | | | | | |

## Claims

1. A compound of formula (I),
(Ar₁)ₘ-(Ar₂)-(X)ₙ (I)
wherein:
Ar₂ is a pyridine, pyrimidine, pyrazine, pyridazine, triazine or tetrazine ring group;
m is from 1 to 5;
n is 0, 1 or 2;
each Ar₁ is independently selected from the following: 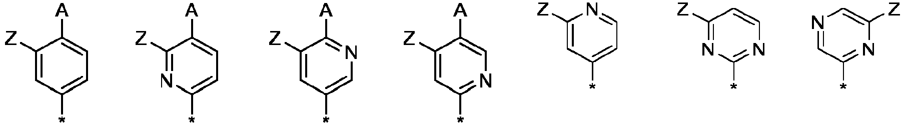 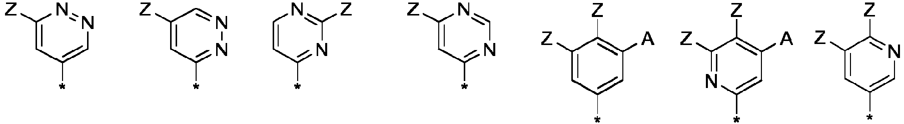 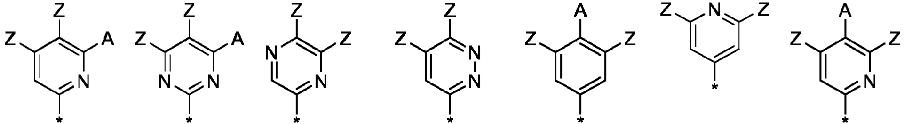 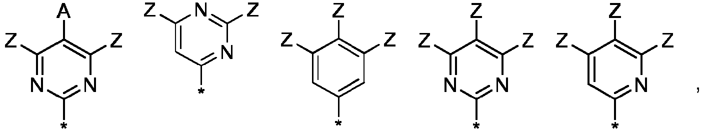
wherein each Z is independently selected from Z2 to Z4: 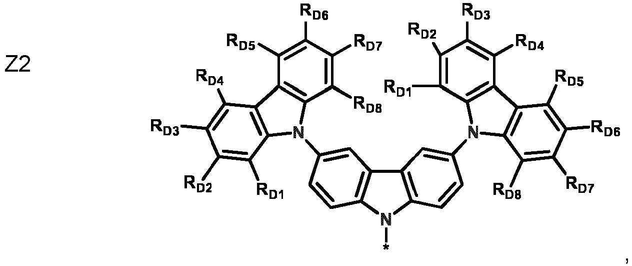 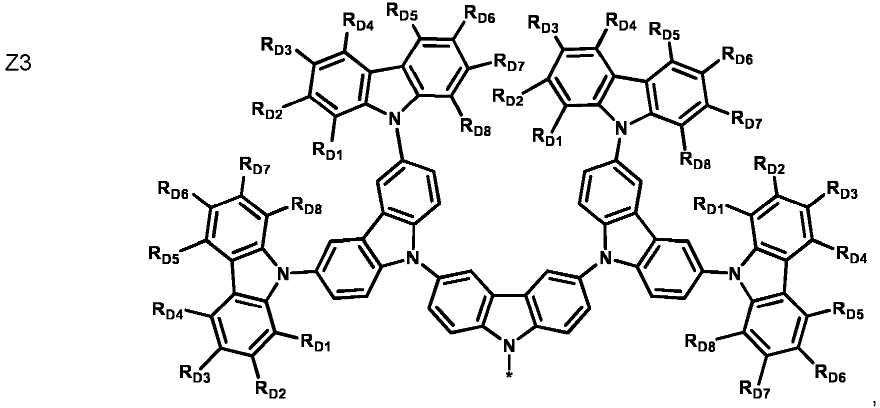 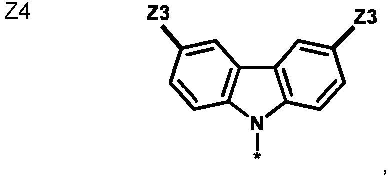
wherein R_{D1}, R_{D4}, R_{D5}, and R_{D8} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, and silyl,
wherein R_{D2}, R_{D3}, R_{D6} and R_{D7} are each independently selected from hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q,
wherein Q is selected from the following: 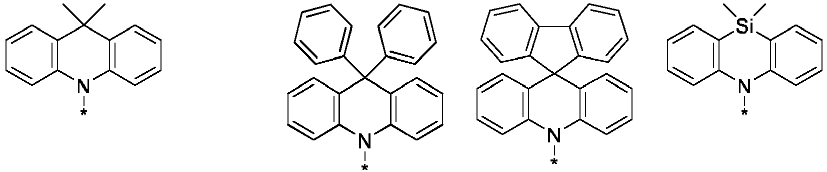 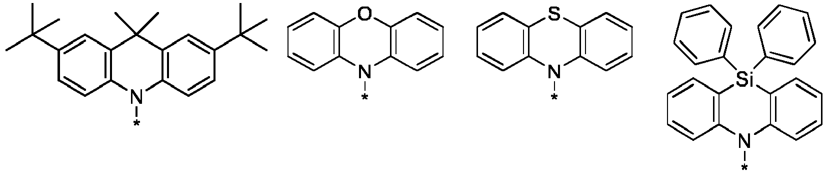 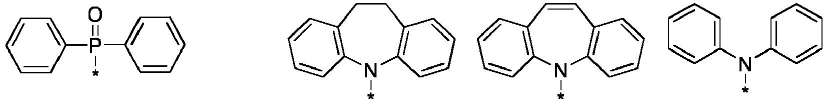 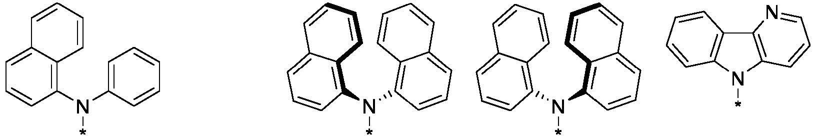 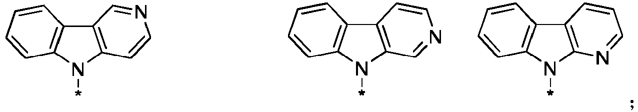
wherein A is selected from hydrogen, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy and silyl;
wherein each X is independently selected from substituted or unsubstituted alkyl, substituted or unsubstituted cycloalkyl, and the following: 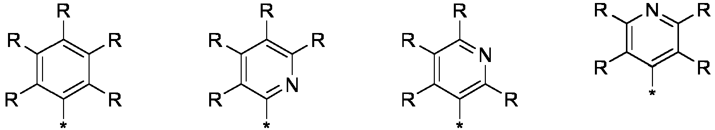 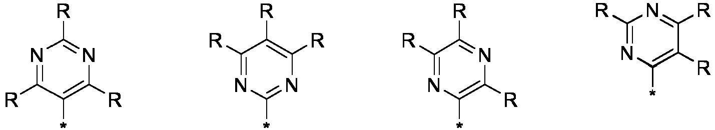 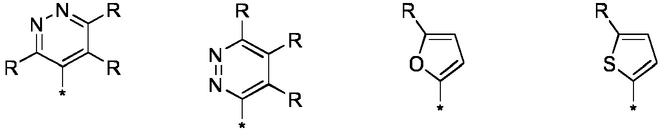 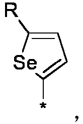
wherein each R is independently selected from the group consisting of hydrogen, deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and diphenylphosphine oxide;
wherein * represents a point of attachment; and
wherein for Z2, each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q.

2. A compound according to claim 1, wherein m is two or more and the compound contains at least two different Ar₁ groups or m is two or more and each of the Ar₁ groups is the same.

3. A compound according to claim 1 or claim 2, wherein for Z2, each of R_{D3} and R_{D6} is independently a substituted or unsubstituted alkyl (more preferably C1-C6 alkyl, even more preferably tertiary butyl); and/or each of R_{D1}, R_{D2}, R_{D4}, R_{D5}, R_{D7} and R_{D8} is hydrogen.

4. A compound according to any one of claims 1 to 3, wherein for Z3 and/or Z4:
(i) each of R_{D3} and R_{D6} is independently selected from deuterium, halo, ester, nitro, cyano, haloalkyl, sulfonyl, carbonyl, substituted or unsubstituted alkyl, substituted or unsubstituted alkoxy, substituted or unsubstituted aryl, substituted or unsubstituted aryloxy, substituted or unsubstituted heteroaryl, substituted or unsubstituted heteroaryloxy, silyl and Q;
(ii) each of R_{D3} and R_{D6} is independently a substituted or unsubstituted alkyl (more preferably C1-C6 alkyl, even more preferably tertiary butyl); and/or
(iii) each of R_{D1}, R_{D2}, R_{D4}, R_{D5}, R_{D7} and R_{D8} is hydrogen.

5. A compound according to any one of claims 1 to 4, wherein A is selected from hydrogen, CN, tetrazole, or a substituted diazole.

6. A compound according to any one of claims 1 to 5, wherein each Z is independently selected from Z2.

7. A compound according to any one of claims 1 to 6, wherein:
(i) the number of Z in formula (I) is 2 or more and each Z is the same or the number of Z in formula (I) is 2 or more and the compound contains at least two different Z dendrons; and/or
(ii) the number of Z in formula (I) is from 2 to 6.

8. A compound according to any one of the preceding claims, wherein m is 2 or 3, and, preferably, n is 0 or 1.

9. A compound according to any one preceding claim, wherein the number of Z dendrons in formula (I) is from two to four, preferably 3 or 4, more preferably 4.

10. A compound according to any one of claims 1 to 9, wherein Ar₂ is a triazine or a tetrazine ring group, optionally wherein:
(i)
each Ar₁ is independently selected from: 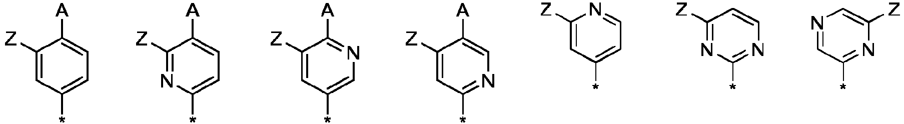 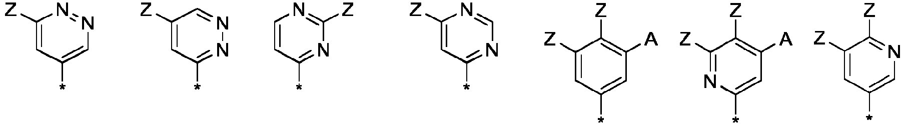 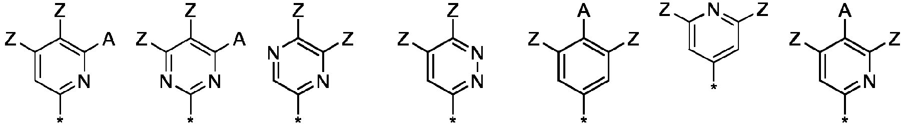 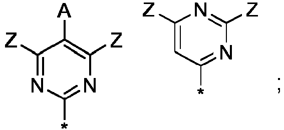 or
(ii) m is 2, and each Ar₁ is independently selected from:

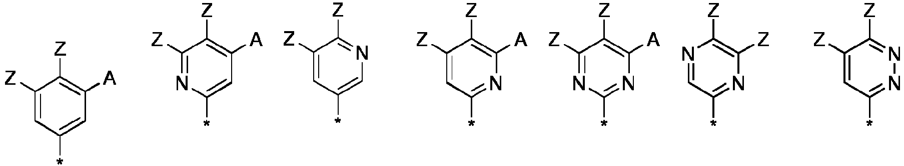

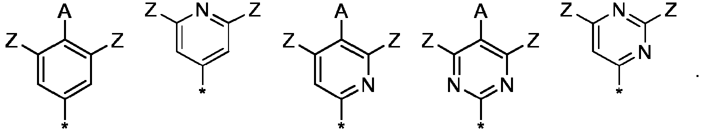

11. A compound according to any one of claims 1 to 8, wherein Ar₂ is a pyridine, pyrimidine, pyrazine or pyridazine ring group, optionally wherein:
(i) each Ar₁ is independently selected from: 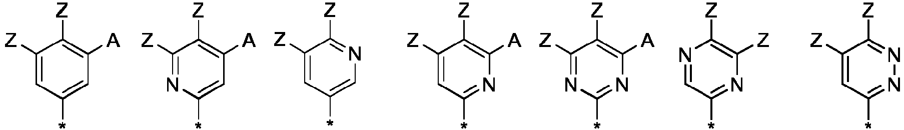 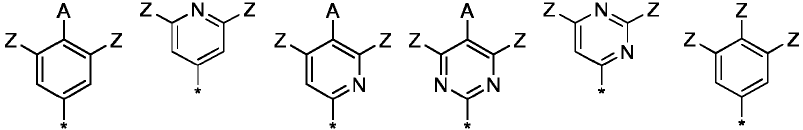 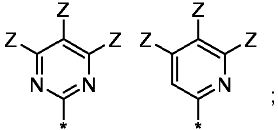 or
(ii) m is 2, and each Ar₁ is independently selected from:

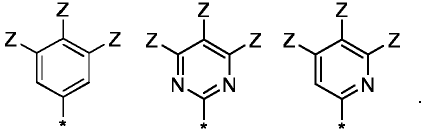

12. A compound according to claim 11, wherein the number of Z in formula (I) is 5 or 6, preferably 6.

13. A compound according to claim 1, selected from the following compounds:
| | |
|---|---|
| I | 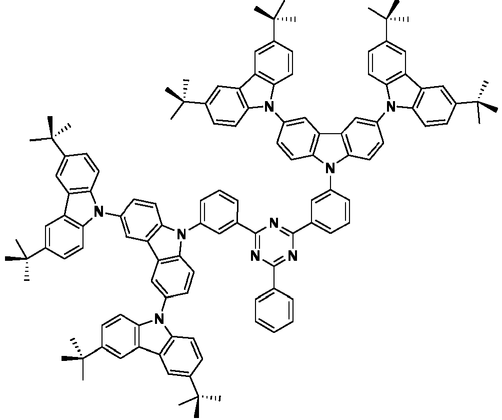 |
| II | 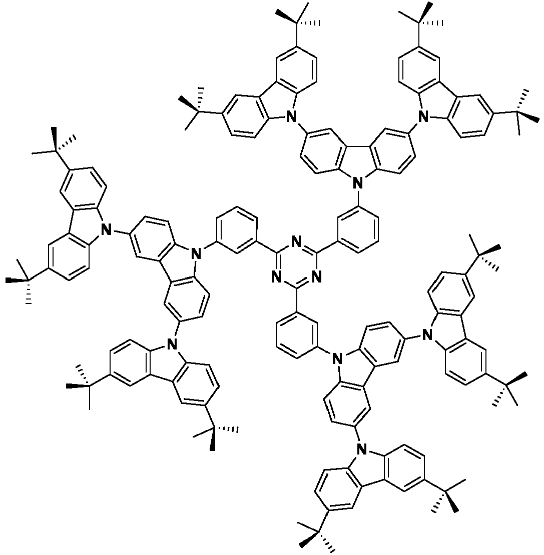 |
| III | 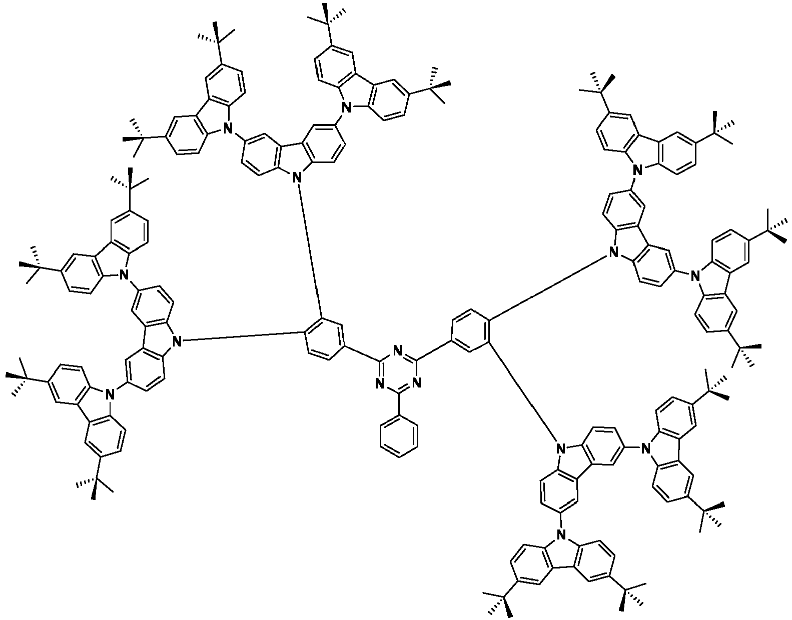 |
| IV | 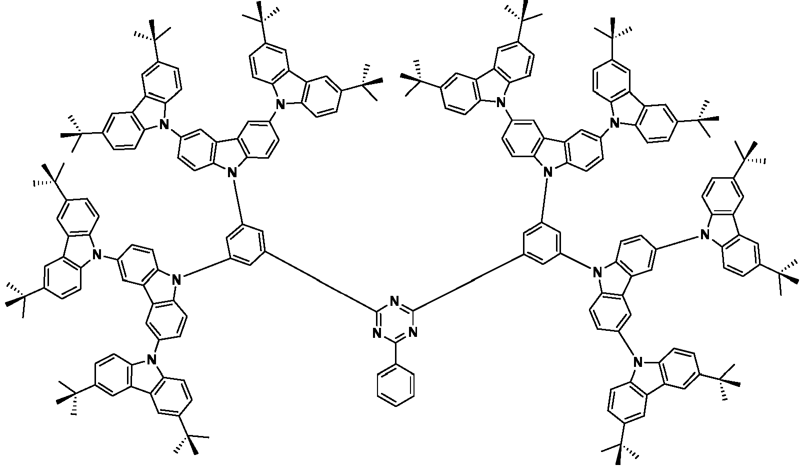 |
| V | 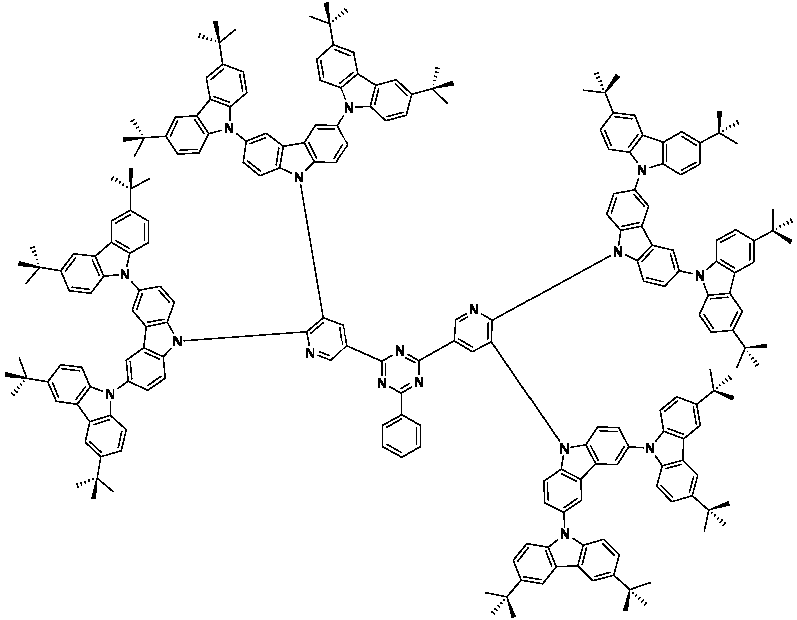 |
| VI | 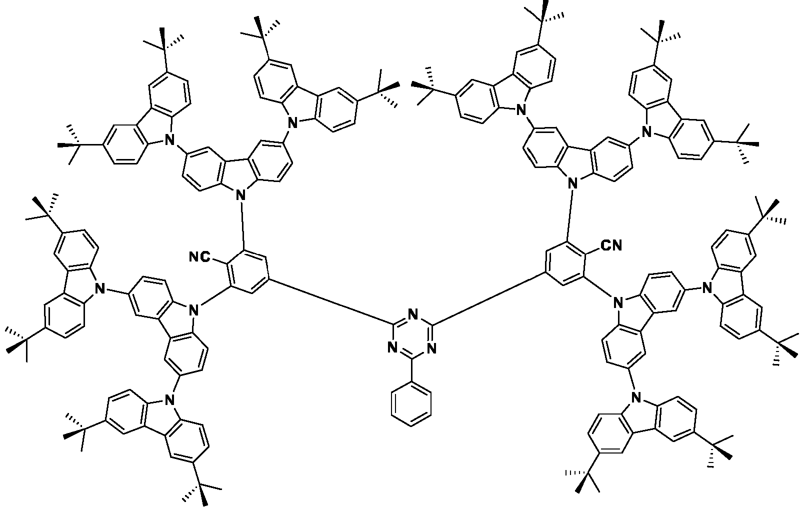 |
| VII | 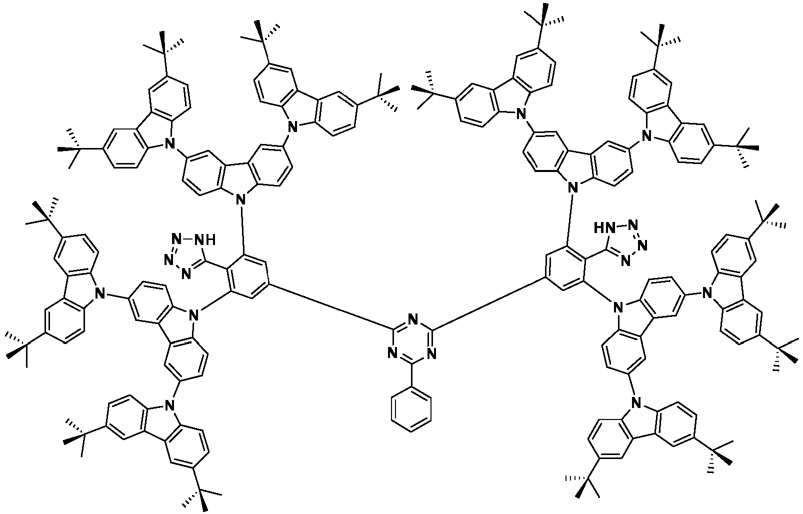 |
| VIII | 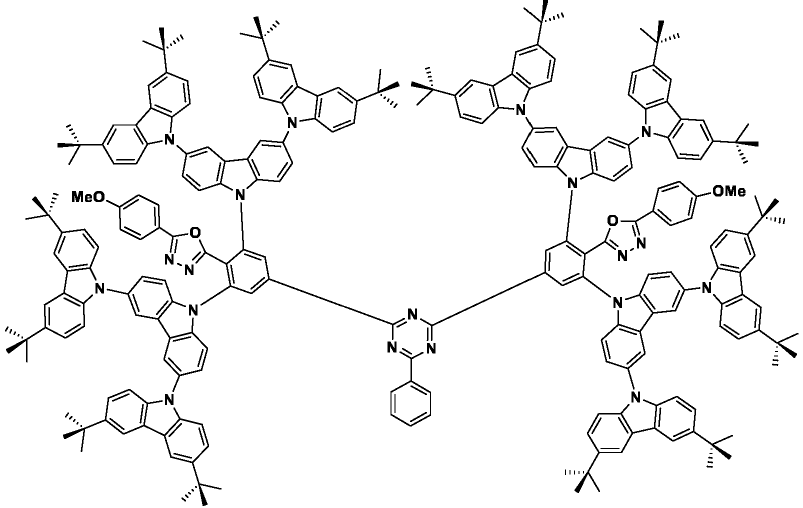 |
| IX | 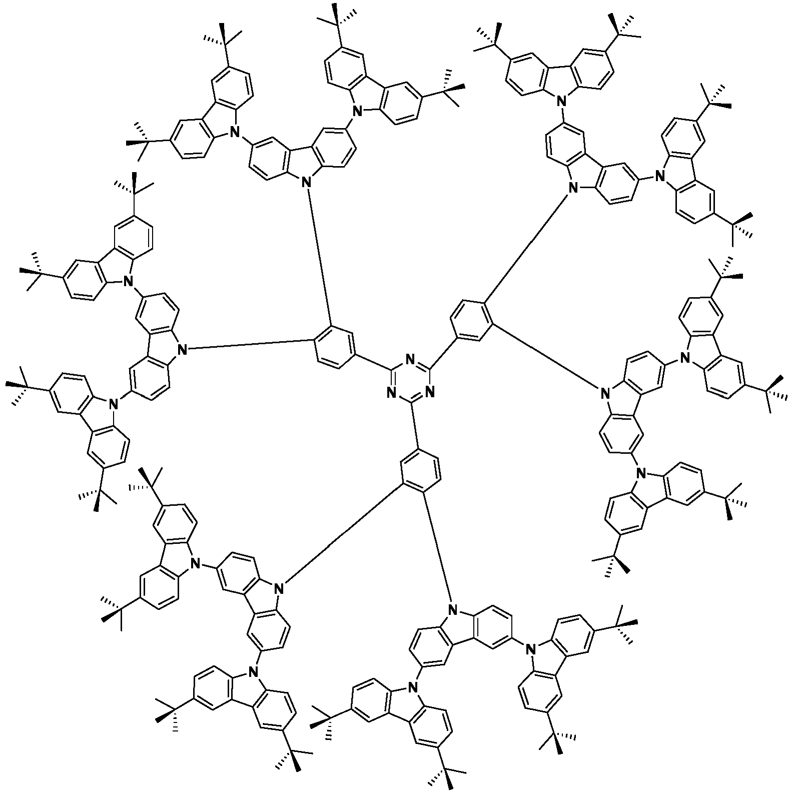 |
| X | 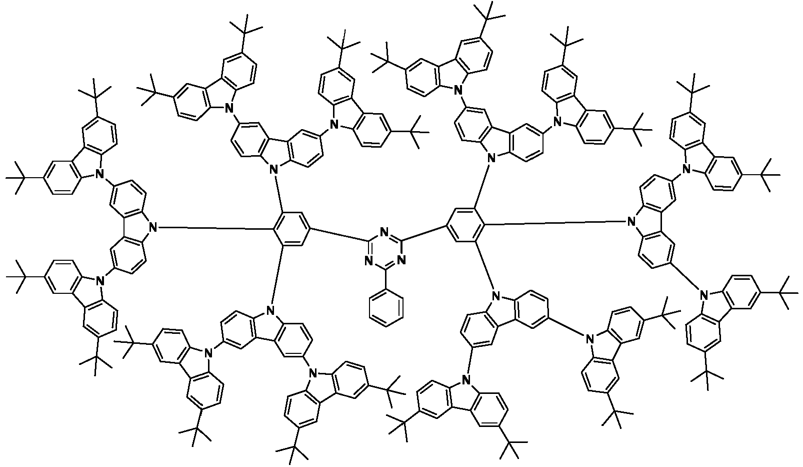 |
| XI | 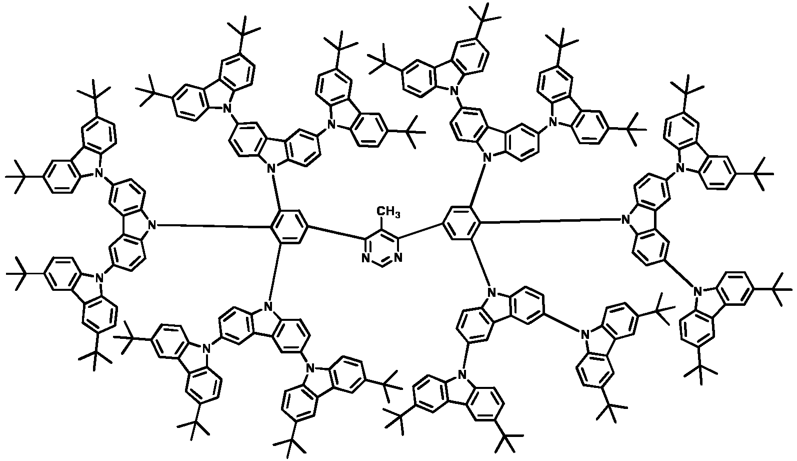 |
| XIII | 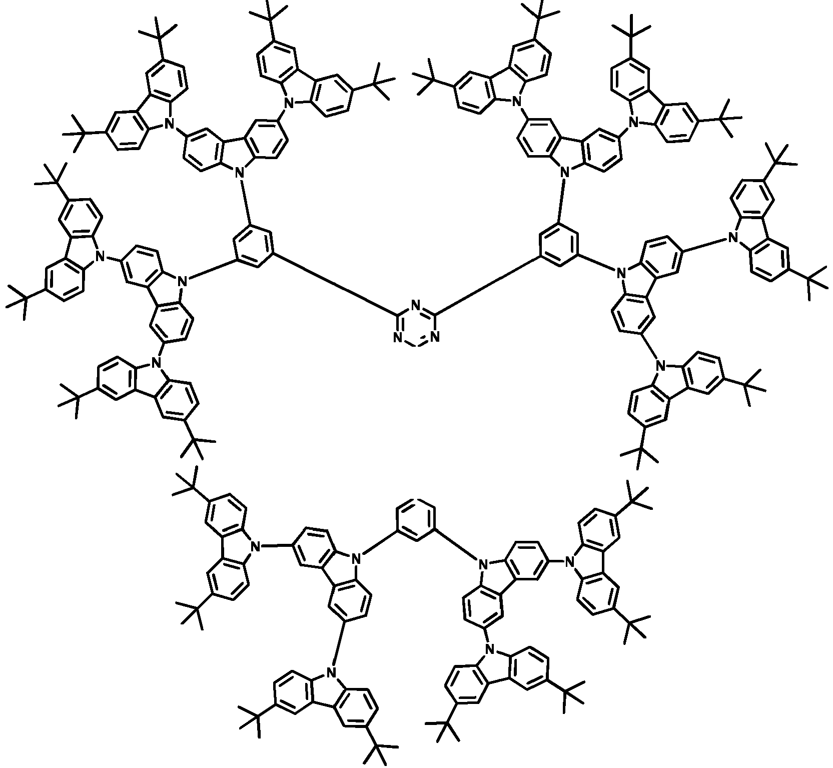 |
| XIV | 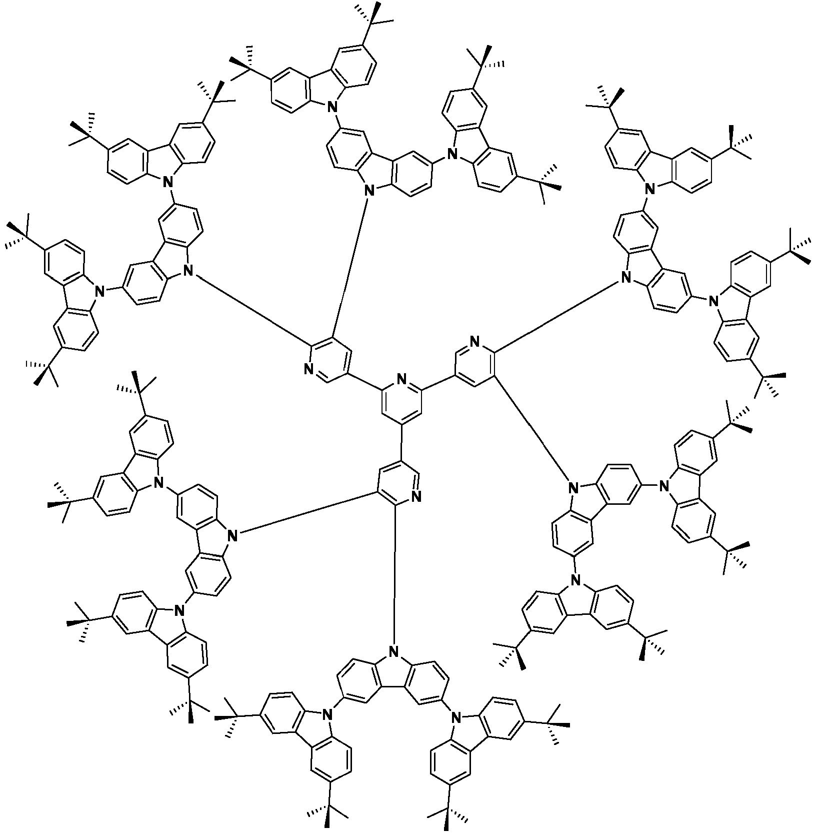 |
| XV | 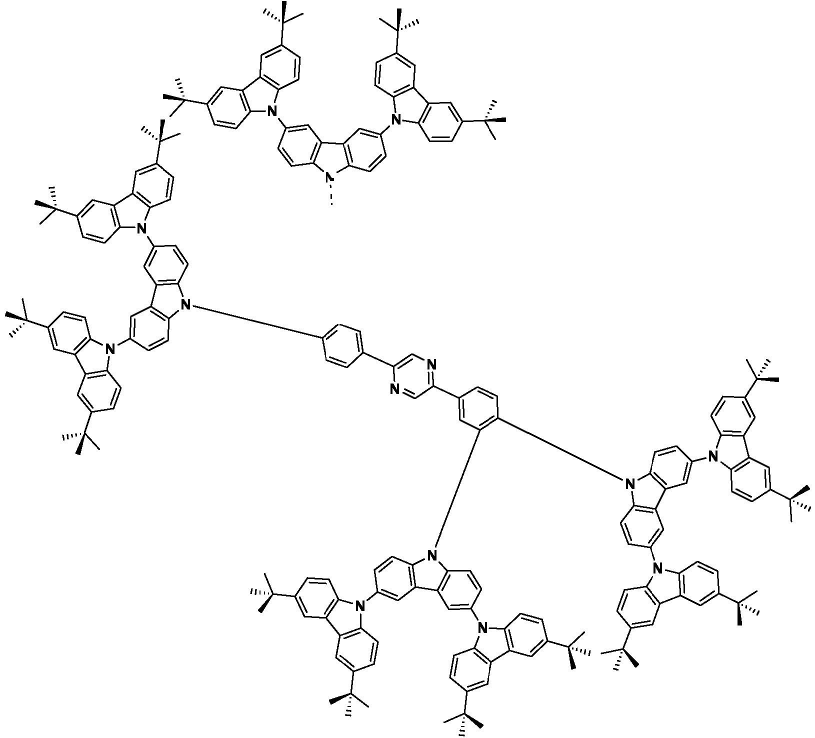 |
| XVI | 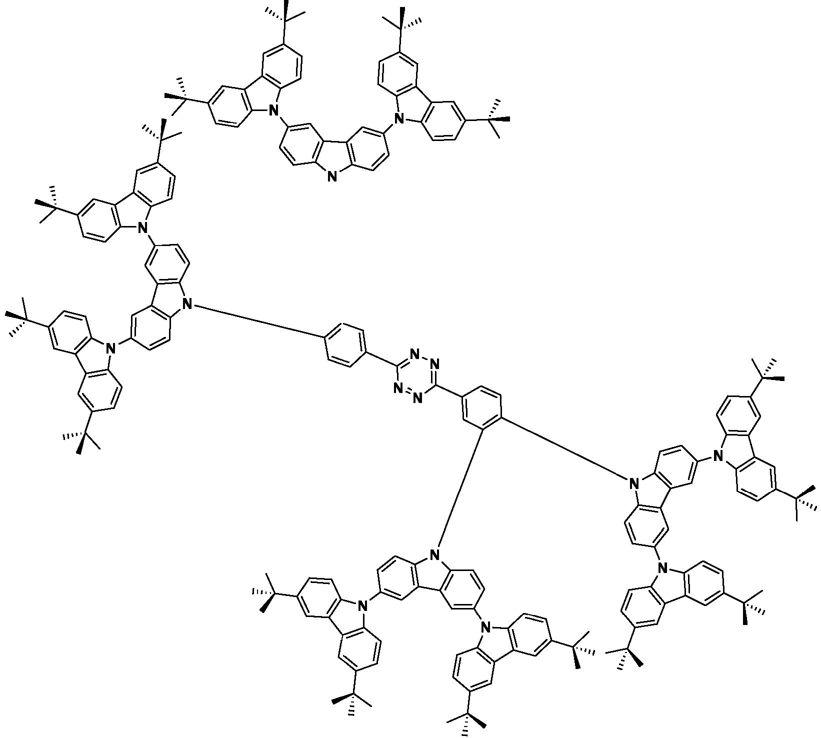 |

14. An organic light emitting device comprising an emissive layer, wherein the emissive layer comprises one or more compounds according to any one of claims 1 to 13, optionally wherein the emissive layer consists essentially of one or more compounds according to any one of claims 1 to 13.

15. A method of preparing an organic light emitting device comprising depositing an emissive layer on a substrate using a solution processing technique, wherein said emissive layer comprises one or more compounds according to any one of claims 1 to 13.

## Patentansprüche

1. Verbindung der Formel (I),
(Ar₁)ₘ-(Ar₂)-(X)ₙ (I)
wobei:
Ar₂ eine Pyridin-, Pyrimidin-, Pyrazin-, Pyridazin-, Triazin- oder Tetrazinringgruppe ist;
m von 1 bis 5 beträgt;
n 0, 1, oder 2 ist;
jedes Ar₁ unabhängig aus Folgendem ausgewählt ist: 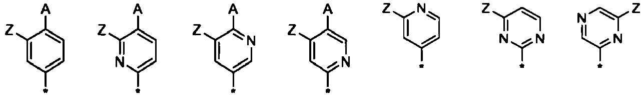 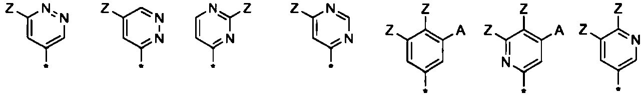 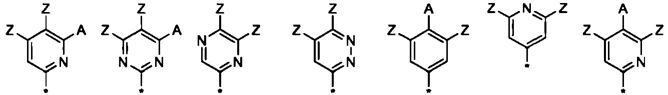 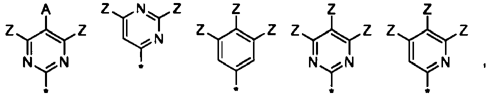
wobei jedes Z unabhängig aus Z2 bis Z4 ausgewählt ist: 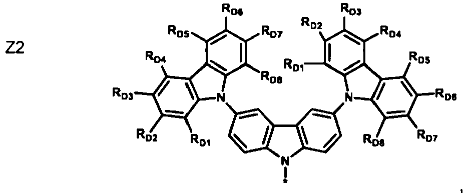 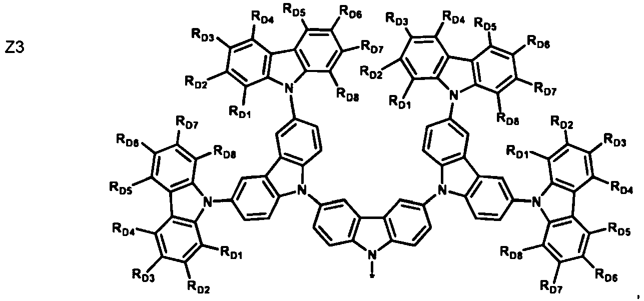 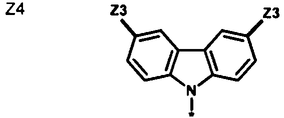
wobei R_{D1}, R_{D4}, R_{D5} und R_{D8} jeweils unabhängig voneinander aus Wasserstoff, Deuterium, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy und Silyl ausgewählt sind,
wobei R_{D2}, R_{D3}, R_{D6} und R_{D7} jeweils unabhängig voneinander aus Wasserstoff, Deuterium, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy, Silyl und Q ausgewählt sind,
wobei Q aus Folgendem ausgewählt ist: 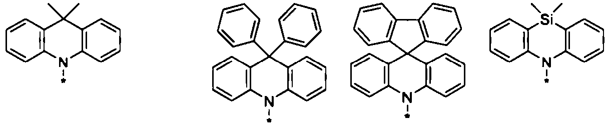 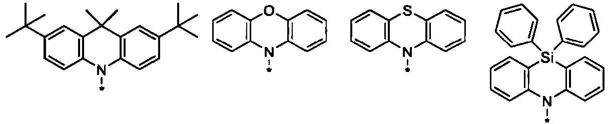 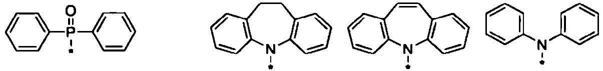 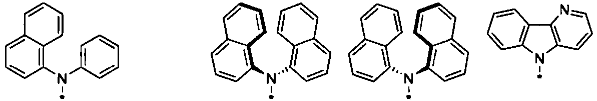 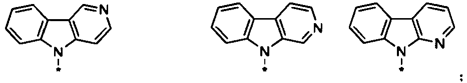
wobei A aus Wasserstoff, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy und Silyl ausgewählt ist;
wobei jedes X unabhängig aus substituiertem oder unsubstituiertem Alkyl, substituiertem oder unsubstituiertem Cycloalkyl und dem Folgenden ausgewählt ist: 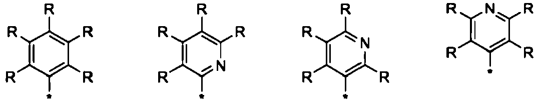 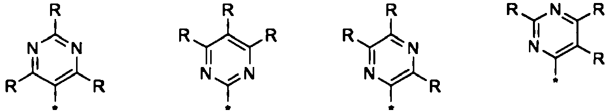 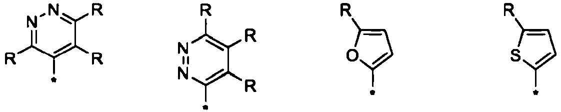 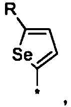
wobei jedes R unabhängig aus der Gruppe ausgewählt ist, die aus Wasserstoff, Deuterium, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy, Silyl und Diphenylphosphinoxid besteht;
wobei * einen Befestigungspunkt darstellt; und
wobei für Z2 jedes von R_{D3} und R_{D6} unabhängig voneinander aus Deuterium, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy, Silyl und Q ausgewählt ist.

2. Verbindung nach Anspruch 1, wobei m zwei oder mehr ist und die Verbindung mindestens zwei verschiedene Ar₁- Gruppen enthält oder m zwei oder mehr ist und jede der Ar₁ -Gruppen gleich ist.

3. Verbindung nach Anspruch 1 oder Anspruch 2, wobei für Z2 jedes von R_{D3} und R_{D6} unabhängig voneinander ein substituiertes oder unsubstituiertes Alkyl (bevorzugter ein C1-C6-Alkyl, noch bevorzugter tertiäres Butyl) ist; und/oder jedes von R_{D1}, R_{D2}, R_{D4}, R_{D5}, R_{D7} und R_{D8} Wasserstoff ist.

4. Verbindung nach einem der Ansprüche 1 bis 3, wobei für Z3 und/oder Z4:
(i) jedes von R_{D3} und R_{D6} unabhängig voneinander aus Deuterium, Halogen, Ester, Nitro, Cyano, Halogenalkyl, Sulfonyl, Carbonyl, substituiertem oder unsubstituiertem Alkyl, substituiertem oder unsubstituiertem Alkoxy, substituiertem oder unsubstituiertem Aryl, substituiertem oder unsubstituiertem Aryloxy, substituiertem oder unsubstituiertem Heteroaryl, substituiertem oder unsubstituiertem Heteroaryloxy, Silyl und Q ausgewählt ist;
(ii) jedes von R_{D3} und R_{D6} unabhängig voneinander ein substituiertes oder unsubstituiertes Alkyl (bevorzugter C1-C6-Alkyl, noch bevorzugter tertiäres Butyl) ist; und/oder
(iii) jedes von R_{D1}, R_{D2}, R_{D4}, R_{D5}, R_{D7} und R_{D8} Wasserstoff ist.

5. Verbindung nach einem der Ansprüche 1 bis 4, wobei A aus Wasserstoff, CN, Tetrazol oder einem substituierten Diazol ausgewählt ist.

6. Verbindung nach einem der Ansprüche 1 bis 5, wobei jedes Z unabhängig aus Z2 ausgewählt ist.

7. Verbindung nach einem der Ansprüche 1 bis 6, wobei:
(i) die Anzahl der Z in Formel (I) 2 oder mehr beträgt und jedes Z das gleiche ist oder die Anzahl der Z in Formel (I) 2 oder mehr beträgt und die Verbindung mindestens zwei unterschiedliche Z-Dendronen enthält; und/oder
(ii) die Anzahl der Z in Formel (I) von 2 bis 6 beträgt.

8. Verbindung nach einem der vorstehenden Ansprüche, wobei m gleich 2 oder 3 und bevorzugt n gleich 0 oder 1 ist.

9. Verbindung nach einem der vorstehenden Ansprüche, wobei die Anzahl der Z-Dendronen in Formel (I) von zwei bis vier, bevorzugt 3 oder 4, bevorzugter 4, beträgt.

10. Verbindung nach einem der Ansprüche 1 bis 9, wobei Ar2 eine Triazin- oder eine Tetrazinringgruppe ist, wahlweise wobei:
(i) jedes Ar₁ unabhängig ausgewählt ist aus: 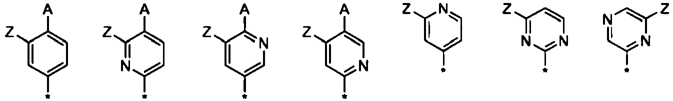 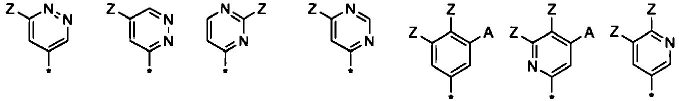 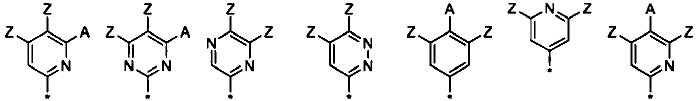 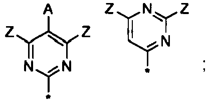 od
(ii) m 2 ist und jedes Ar₁ unabhängig ausgewählt ist aus:

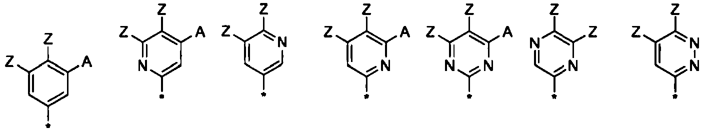

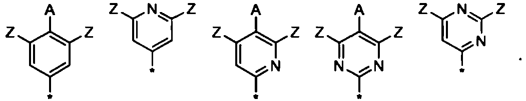

11. Verbindung nach einem der Ansprüche 1 bis 8, wobei Ar₂ eine Pyridin-, Pyrimidin-, Pyrazin- oder Pyridazinringgruppe ist, wahlweise wobei:
(i) jedes Ar₁ unabhängig ausgewählt ist aus: 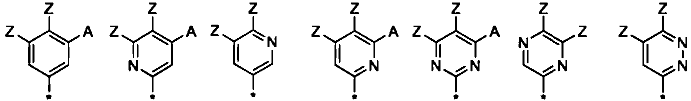 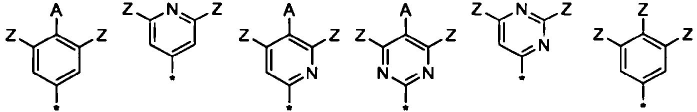 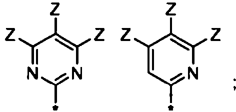 od
(ii) m 2 ist und jedes Ar₁ unabhängig ausgewählt ist aus:

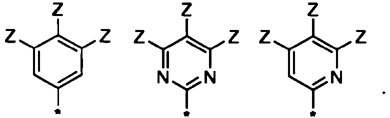

12. Verbindung nach Anspruch 11, wobei die Anzahl der Z in Formel (I) 5 oder 6, bevorzugt 6, beträgt.

13. Verbindung nach Anspruch 1, ausgewählt aus den folgenden Verbindungen:
| | |
|---|---|
| I | 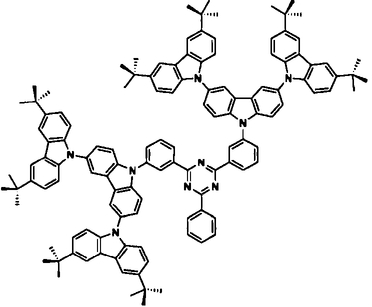 |
| II | 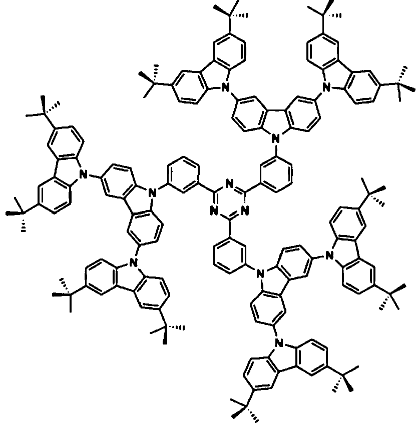 |
| III | 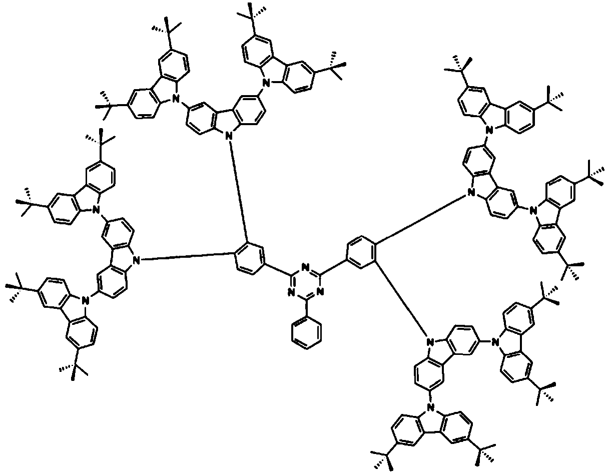 |
| IV | 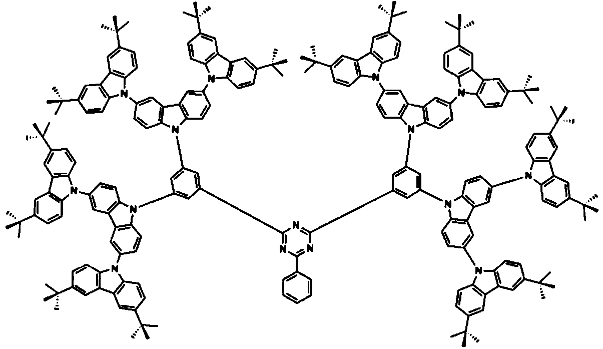 |
| V | 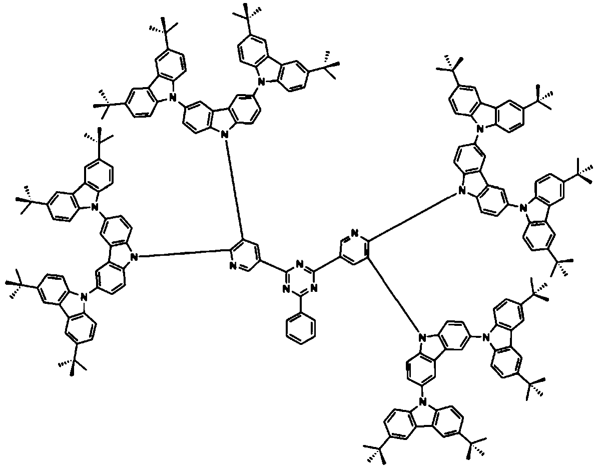 |
| VI | 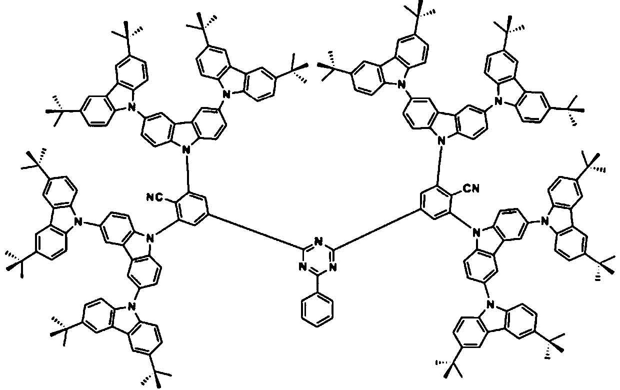 |
| VII | 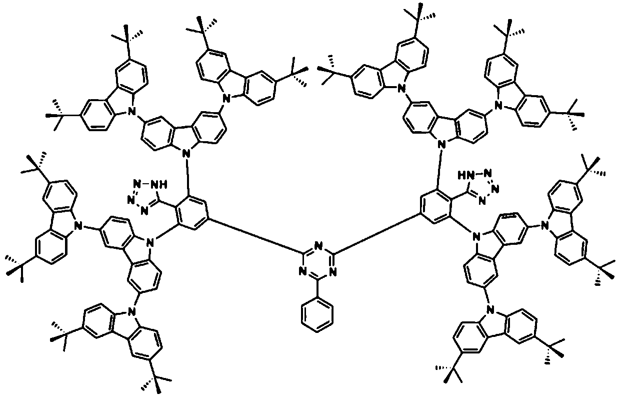 |
| VIII | 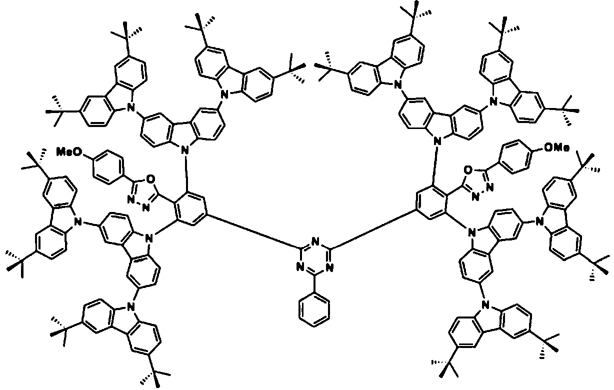 |
| IX | 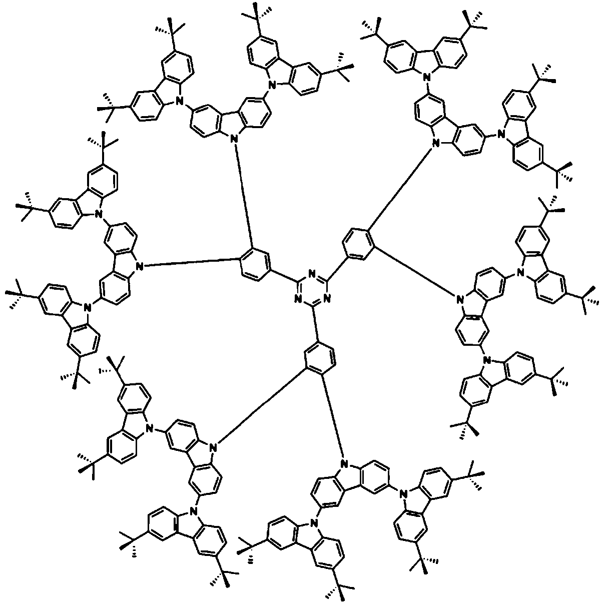 |
| X | 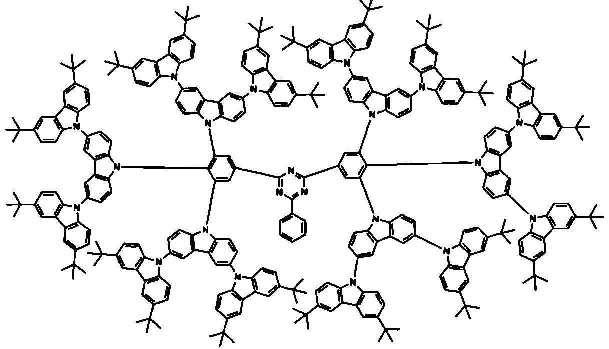 |
| XI | 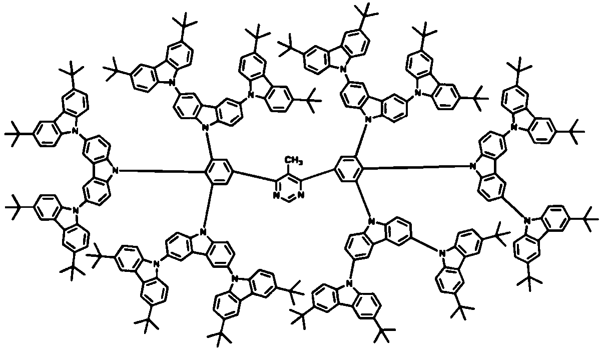 |
| XIII | 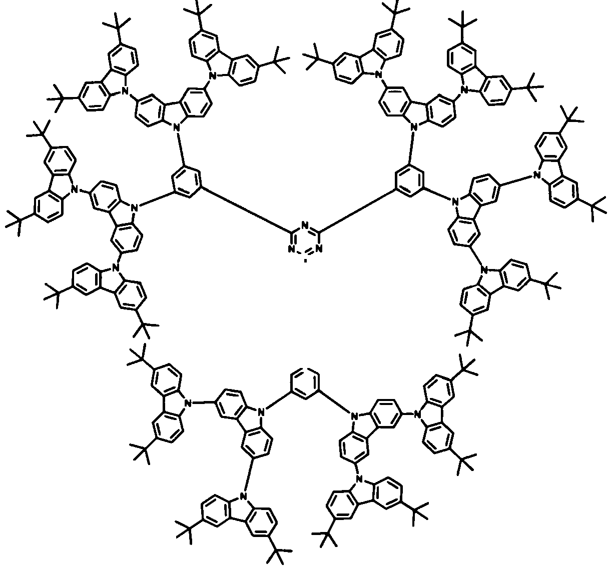 |
| | |
|---|---|
| XIV | 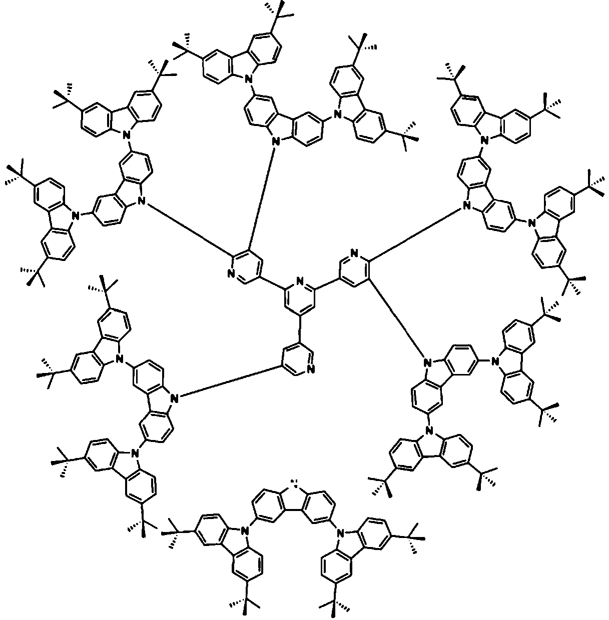 |
| | |
|---|---|
| XV | 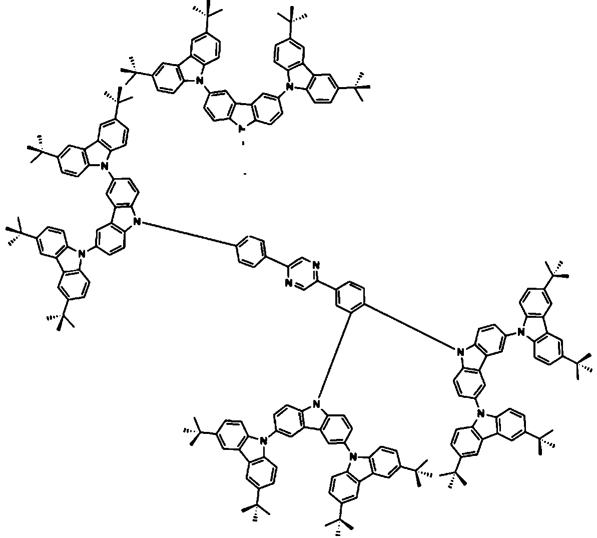 |
| | |
|---|---|
| XVI | 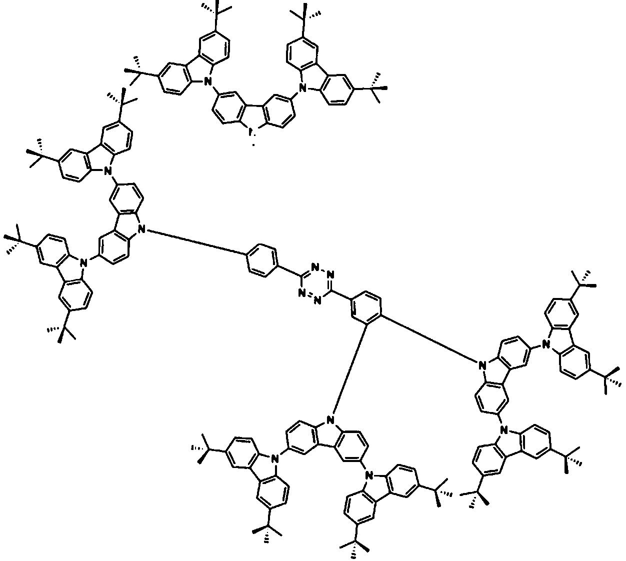 |

14. Organische Licht emittierende Vorrichtung, die eine emittierende Schicht umfasst, wobei die emittierende Schicht eine oder mehrere Verbindungen nach einem der Ansprüche 1 bis 13 umfasst, wahlweise wobei die emittierende Schicht im Wesentlichen aus einer oder mehreren Verbindungen nach einem der Ansprüche 1 bis 13 besteht.

15. Verfahren zur Herstellung einer organischen Licht emittierenden Vorrichtung, die Aufbringen einer emittierenden Schicht auf ein Substrat unter Verwendung einer Lösungsverarbeitungstechnik umfasst, wobei die emittierende Schicht eine oder mehrere Verbindungen nach einem der Ansprüche 1 bis 13 umfasst.

## Revendications

1. Composé de la formule (I),
(Ar₁)ₘ-(Ar₂)-(X)ₙ (I)
dans lequel :
Ar₂ est un groupe cyclique pyridine, pyrimidine, pyrazine, pyridazine, triazine ou tétrazine ;
m est de 1 à 5 ;
n est de 0, 1 ou 2 ;
chaque Ar₁ est sélectionné indépendamment parmi les suivants : 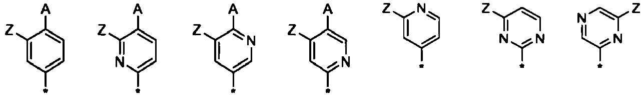 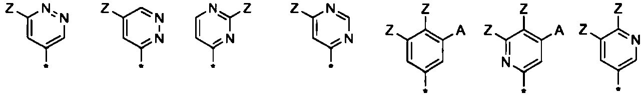 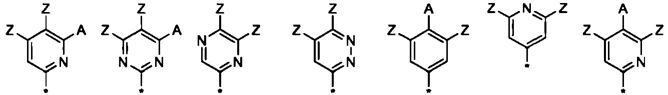 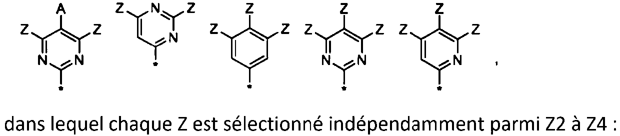 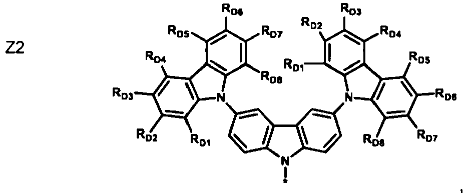 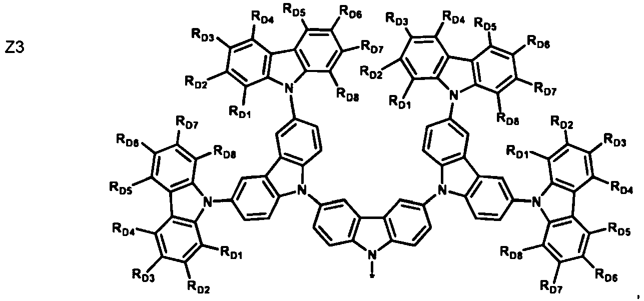 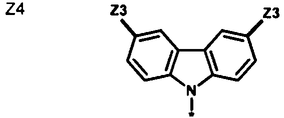
dans lequel R_{D1}, R_{D4}, R_{D5} et R_{D8} sont chacun sélectionnés indépendamment parmi hydrogène, deutérium, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué et silyle,
dans lequel R_{D2}, R_{D3}, R_{D6} et R_{D7} sont chacun sélectionnés indépendamment parmi hydrogène, deutérium, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué, silyle et Q,
dans lequel Q est sélectionné parmi les suivants : 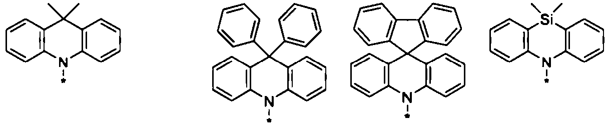 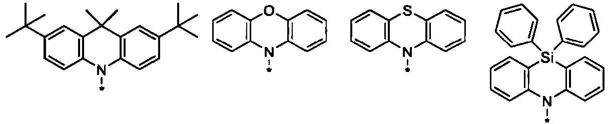 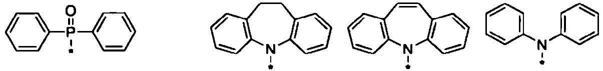 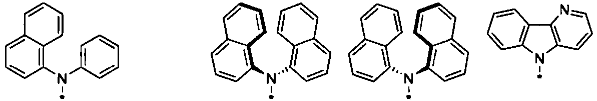 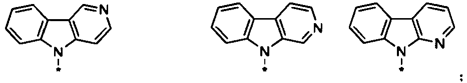
dans lequel A est sélectionné parmi hydrogène, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué et silyle ;
dans lequel chaque X est sélectionné indépendamment parmi alkyle substitué ou non substitué, cycloalkyle substitué ou non substitué, et les suivants : 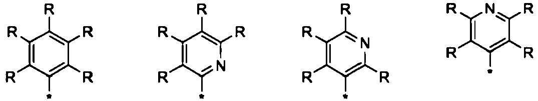 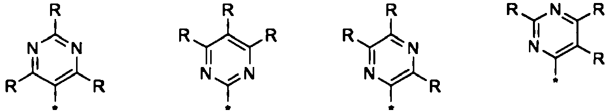 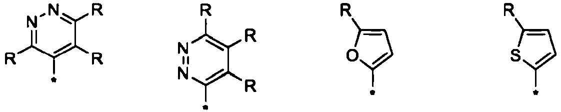 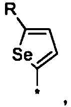
dans lequel R est sélectionné indépendamment à partir du groupe constitué par hydrogène, deutérium, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué, silyle et oxyde de diphénylphosphine ;
dans lequel * représente un point d'attache ; et
dans lequel pour Z2, chacun parmi R_{D3} et R_{D6} est sélectionné indépendamment parmi deutérium, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué, silyle et Q.

2. Composé selon la revendication 1, dans lequel m est deux ou plus et le composé contient au moins deux groupes Ar₁ différents ou m est deux ou plus et chacun parmi les groupes Ar₁ est le même.

3. Composé selon la revendication 1 ou la revendication 2, dans lequel pour Z2, chacun parmi R_{D3} et R_{D6} est indépendamment un alkyle substitué ou non substitué (plus préférentiellement un alkyle en C1-C6, encore plus préférentiellement un tertio-butyle) ; et/ou chacun parmi R_{D1}, R_{D2}, R_{D4}, R_{D5}, R_{D7} et R_{D8} est hydrogène.

4. Composé selon l'une quelconque des revendications 1 à 3, dans lequel pour Z3 et/ou Z4 :
(i) chacun parmi R_{D3} et R_{D6} est sélectionné indépendamment parmi deutérium, halo, ester, nitro, cyano, haloalkyle, sulfonyle, carbonyle, alkyle substitué ou non substitué, alcoxy substitué ou non substitué, aryle substitué ou non substitué, aryloxy substitué ou non substitué, hétéroaryle substitué ou non substitué, hétéroaryloxy substitué ou non substitué, silyle et Q;
(ii) chacun parmi R_{D3} et R_{D6} est indépendamment un alkyle substitué ou non substitué (plus préférentiellement un alkyle en C1-C6, encore plus préférentiellement un tertio-butyle) ; et/ou
(iii) chacun parmi R_{D1}, R_{D2}, R_{D4}, R_{D5}, R_{D7} et R_{D8} est hydrogène.

5. Composé selon l'une quelconque des revendications 1 à 4, dans lequel A est sélectionné parmi hydrogène, CN, tétrazole ou un diazole substitué.

6. Composé selon l'une quelconque des revendications 1 à 5, dans lequel chaque Z est sélectionné indépendamment à partir de Z2.

7. Composé selon l'une quelconque des revendications 1 à 6, dans lequel :
(i) le nombre de Z dans la formule (I) est de 2 ou plus et chaque Z est le même ou le nombre de Z dans la formule (I) est de 2 ou plus et le composé contient au moins deux dendrons Z différents ; et/ou
(ii) le nombre de Z dans la formule (I) est de 2 à 6.

8. Composé selon l'une quelconque des revendications précédentes, dans lequel m est de 2 ou 3, et, de préférence, n est de 0 ou 1.

9. Composé selon l'une quelconque des revendications précédentes, dans lequel le nombre de dendrons Z dans la formule (I) est de deux à quatre, de préférence 3 ou 4, plus préférentiellement 4.

10. Composé selon l'une quelconque des revendications 1 à 9, dans lequel Ar₂ est un groupe cyclique triazine ou tétrazine, facultativement dans lequel :
(i) chaque Ar₁ est sélectionné indépendamment parmi : 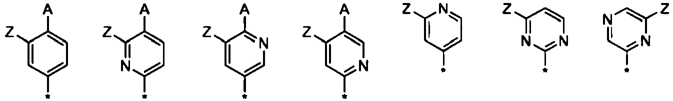 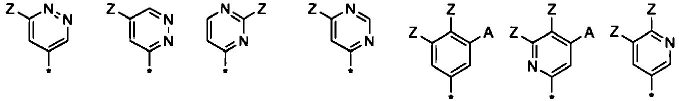 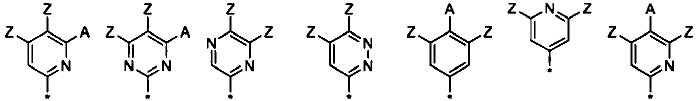 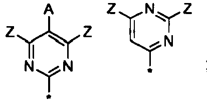 ou
(ii) m est de 2, et chaque Ar₁ est sélectionné indépendamment parmi :

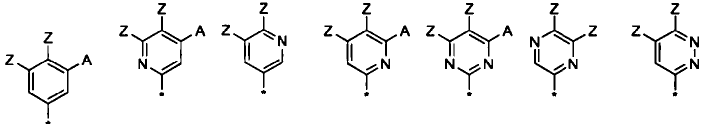

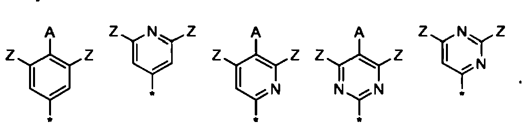

11. Composé selon l'une quelconque des revendications 1 à 8, dans lequel Ar₂ est un groupe cyclique pyridine, pyrimidine, pyrazine ou pyridazine, facultativement dans lequel :
(i) chaque Ar₁ est sélectionné indépendamment parmi : 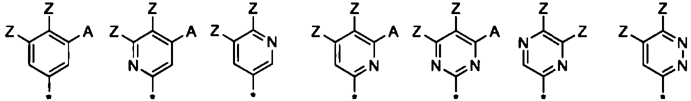 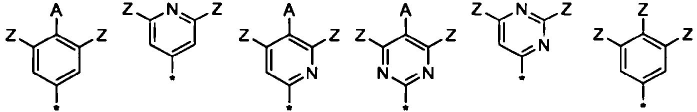 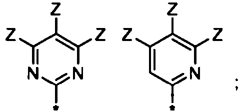 ou
(ii) m est de 2, et chaque Ar₁ est sélectionné indépendamment parmi :

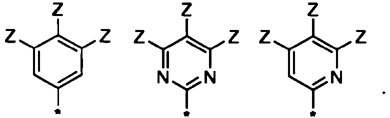

12. Composé selon la revendication 11, dans lequel le nombre de Z dans la formule (I) est de 5 ou 6, de préférence de 6.

13. Composé selon la revendication 1, sélectionné parmi les composés suivants :
| | |
|---|---|
| I | 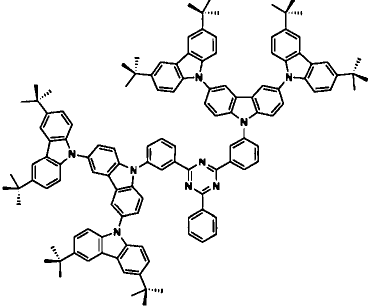 |
| II | 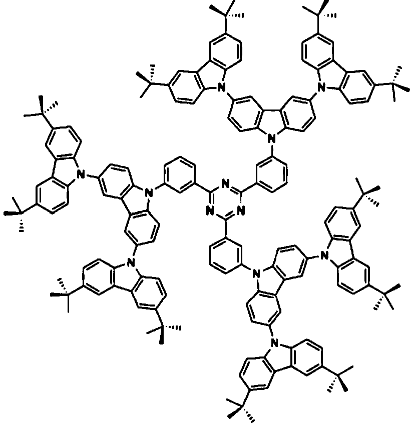 |
| III | 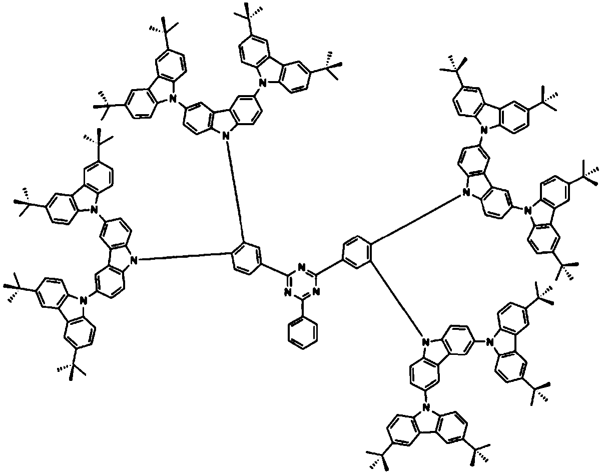 |
| IV | 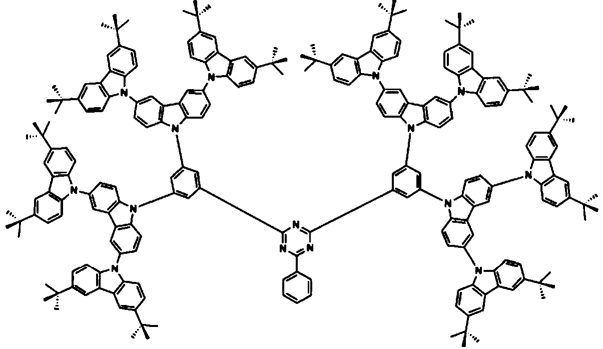 |
| V | 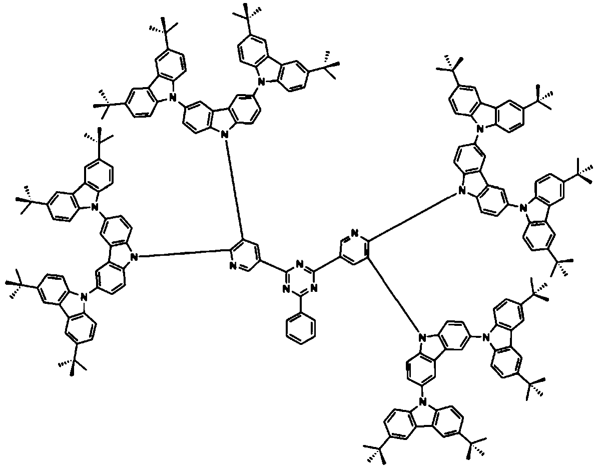 |
| VI | 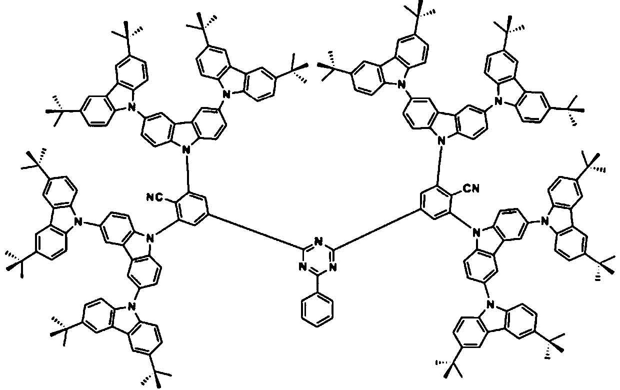 |
| VII | 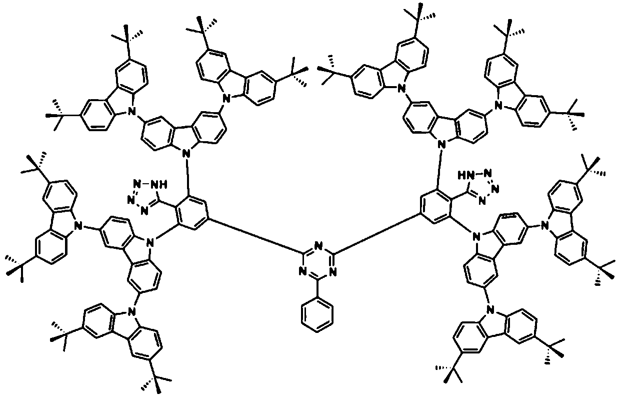 |
| VIII | 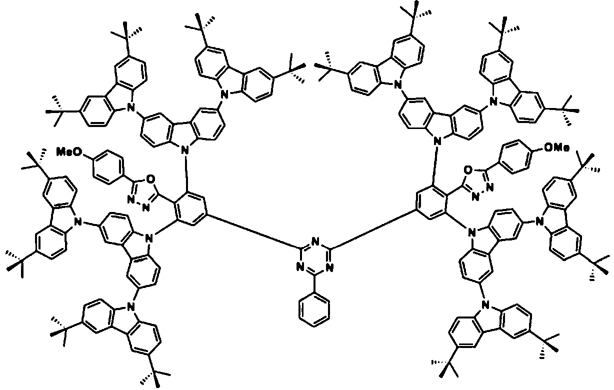 |
| IX | 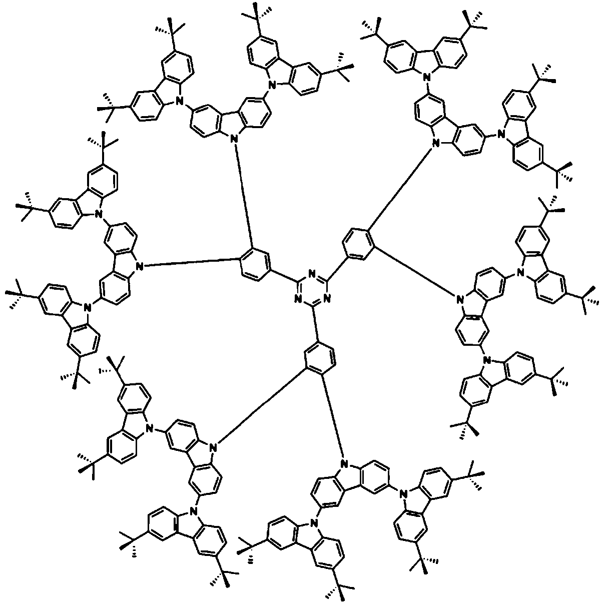 |
| X | 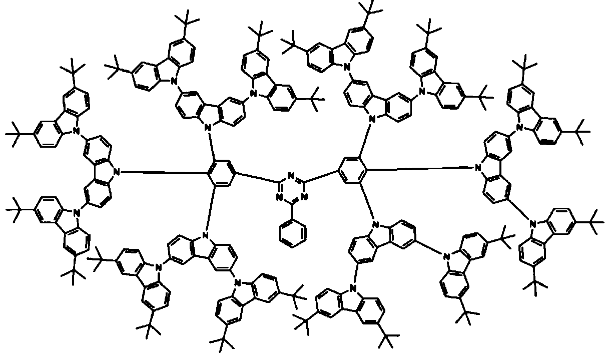 |
| XI | 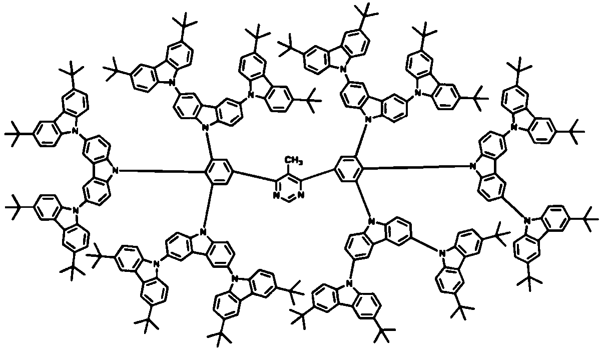 |
| XIII | 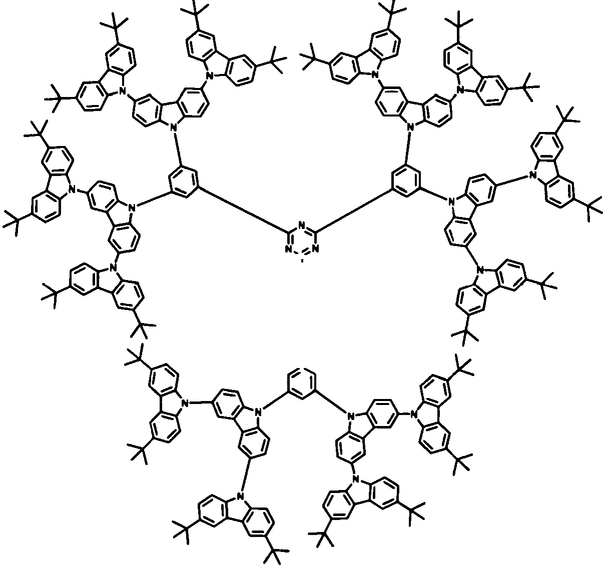 |
| | |
|---|---|
| XIV | 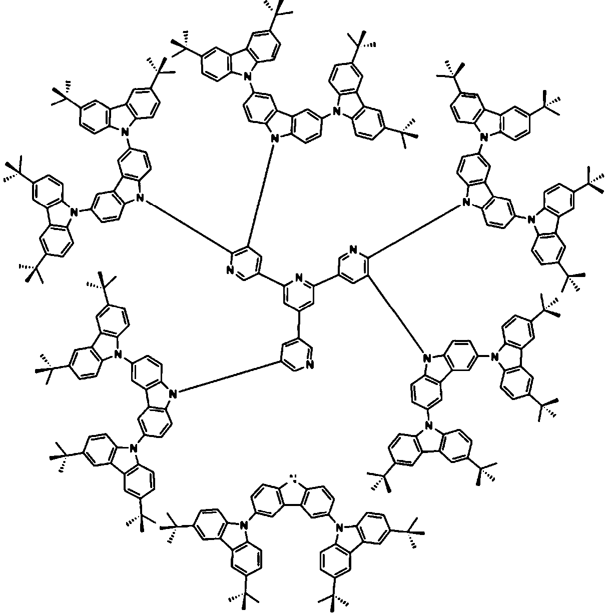 |
| | |
|---|---|
| XV | 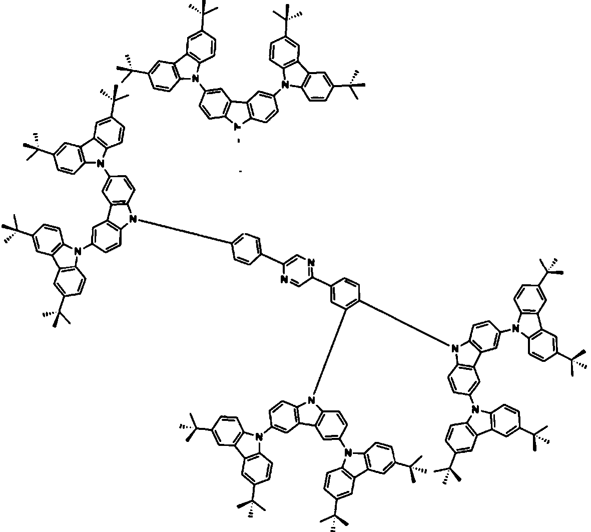 |
| | |
|---|---|
| XVI | 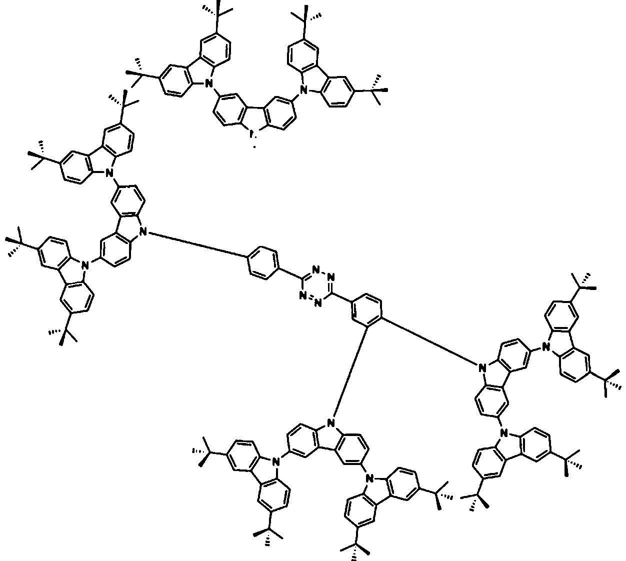 |

14. Dispositif émetteur de lumière organique comprenant une couche émissive, dans lequel la couche émissive comprend un ou plusieurs composés selon l'une quelconque des revendications 1 à 13, et facultativement dans lequel la couche émissive est constituée essentiellement d'un ou de plusieurs composés selon l'une quelconque des revendications 1 à 13.

15. Procédé de préparation d'un dispositif émetteur de lumière organique comprenant le dépôt d'une couche émissive sur un substrat en utilisant une technique de traitement en solution, dans lequel ladite couche émissive comprend un ou plusieurs composés selon l'une quelconque des revendications 1 à 13.
